# EUROPÄISCHE PATENTSCHRIFT

(11) **EP 3 695 906 B1**
(45) Veröffentlichungstag und Bekanntmachung des Hinweises auf die Patenterteilung: **02.08.2023**
(21) Anmeldenummer: 20157443.1
(22) Anmeldetag: 14.02.2020
(51) Int. Cl.: B01L 3/02, C12N 15/10, C12Q 1/6806, A61C 15/02

(54) **PROBENTRANSFERWERKZEUG**
SAMPLE TRANSFER TOOL
OUTIL DE TRANSFERT D'ÉCHANTILLONS

(30) Priorität: 14.02.2019 DE 102019201966
(43) Veröffentlichungstag der Anmeldung: 19.08.2020
(73) Patentinhaber: AXAGARIUS GmbH & Co. KG, 52355 Düren (DE)
(72) Erfinder: Kirsch, Dr. Christoph, 50259 Pulheim (DE); Yaroshevskaya, Elena, 50126 Bergheim (DE)
(74) Vertreter: dompatent von Kreisler Selting Werner - Partnerschaft von Patent- und Rechtsanwälten mbB

(56) Entgegenhaltungen:
- EP-A2- 0 989 192
- EP-A2- 1 069 190
- WO-A1-2016/169679
- WO-A1-2017/027538
- US-A1- 2002 012 928
- Anonymous: "Blutentnahmesysteme für die Intensivmedizin. Ergänzung zum Blutgassystem", Roche, 1. Januar 2011 (2011-01-01), Seiten 1-8, XP55679008, Gefunden im Internet: URL:https://www.roche.de/res/content/7687/ blutentnahmesysteme_bga.pdf [gefunden am 2020-03-24]

## Beschreibung

Die Erfindung betrifft die Verwendung eines mit Wirkstoffen beschichteten, stabförmigen Probentransferwerkzeugs zum Transfer von Nukleinsäure-haltigen Probenmaterial in Reaktionsgemische von enzymatischen Nachweisreaktionen.

### Hintergrund der Erfindung

Bei direkt-PCR Nachweisverfahren für Vollblut sowie für Pflanzenmaterial haben sich gegenwärtig zwei Verfahren für die Probenvorbehandlung etabliert:
Ein erstes Verfahren mit einfacher Probenvorbehandlung, wie z.B. Verdünnung der Probe oder ein schneller Aufschluss des Blutes in einem Lysepuffer, teilweise kombiniert mit Proteinase und/oder Inkubationsschritten bei erhöhten Temperaturen. Diese Verfahren erfordern typischerweise für die Probenvorbereitung mindestens ein Reaktionsgefäß, Lysepuffer, ggf. Neutralisierungspuffer und Proteinase, Inkubationsschritte bei erhöhten Temperaturen von 5 bis 30 min sowie Pipettenspitzen zum Transfer der Lösungen.

Ein zweites, "echtes" direkt-PCR Verfahren, in der ein Blutaliquot direkt als Template in die PCR-Reaktion gegeben wird. Hierbei werden Blutvolumen von 0,2 bis 5 µl für PCR-Ansätze von 10 bis 50 µl empfohlen.

Das erste Verfahren hat den Vorteil, dass im Blut befindliche PCR-Inhibitoren durch die Lyse zerstört oder inaktiviert werden können, so dass die nachfolgende PCR zuverlässiger ablaufen kann, bzw. die Anforderungen an die Robustheit der PCR Reagentien geringer sind. Es hat jedoch den Nachteil, dass mehr Schritte, Materialien und Zeit nötig sind, als für das zweite Verfahren.

Das zweite Verfahren hat den Vorteil, dass die Handhabungsschritte weitestgehend minimiert sind, hat jedoch den Nachteil, dass die Anforderungen an die Robustheit des PCR-Systems wesentlich höher sind, da PCR-Inhibitoren in recht großer Menge in die PCR eingetragen werden, da erstens das Blutvolumen, welches in die PCR gelangt, typischerweise höher ist als im ersten Verfahren und da zweitens Inhibitoren im Blut in keiner Weise vor Beginn der PCR inaktiviert, zurückgehalten oder abgetrennt werden. Im zweiten Verfahren wird also lediglich eine Probenmenge in die PCR getragen, ohne dass die Probe in irgendeiner Weise eine Behandlung erfährt, die über den Transfer in die PCR hinausgeht.

Des Weiteren hat das zweite Verfahren den Nachteil, dass bei Einsatz von typischerweise 1 µl Blut je 20 µl PCR die Proteine des Blutes während der PCR ausfallen, die PCR deutlich verfärben und nach der Reaktion bzw. vor der Gelelektrophorese typsicherweise eine Behandlung erforderlich ist, wie z.B. Zentrifugation der Probe oder Zugabe von Proteinase zwecks Entfernung der Trübstoffe und somit Vorbereitung der Probe für die DNA-Gelelektrophorese. Andernfalls behindern oder vereiteln die Trübstoffe sogar die gelelektrophoretische Analyse der PCR-Amplifikate.

Weiterhin ist für das zweite Verfahren nachteilig, dass, trotz großer Robustheit diverser PCR-Systeme, die DNA-Amplifikation von einigen Bluttypen (organismenabhängig) dennoch unzureichend funktioniert und/oder die Zugabe einer sehr kleinen (deutlich unter 1 µl) Blutmenge erfordert, insbesonders wenn man sich beim PCR-Volumen auf 10 µl beschränkt und nicht, wie bei einigen Verfahren des Standes der Technik, 50 µl Reaktionen durchführt.

Erfahrungsgemäß lassen sich Blutproben von weniger als 1 µl jedoch nur schwer handhaben, erfordern spezielle Pipettenspitzen für das Pipettieren von Submikoliter-Proben und erfordern mehr Erfahrung und Aufmerksamkeit durch den Anwender als beim Pipettieren von Mikoliterproben.

Die Aufgabe der vorliegenden Erfindung bestand nun darin, die Vorteile des ersten Verfahrens (Inaktivierung von im Blut befindlichen PCR-Inhibitoren) und des zweiten Verfahrens (direkte Zugabe von Blut als Template in die PCR) zu verbinden, jedoch ohne die jeweiligen nachteiligen Nebeneffekte in Kauf zu nehmen. Dabei sollten unterschiedliche Körperflüssigkeiten, einschließlich Vollblute, mit verschiedenen Antikoagulantien (EDTA, Citrat, Heparin) wie auch verschiedene Organismen (Mensch, Maus, Ratte, Kaninchen, Meerschweinchen, Huhn) berücksichtigt werden. Weiterhin ist zu berücksichtigen, dass, aus Kostengründen, die PCR in einem möglichst kleinen Volumen zu etablieren ist, welches jedoch groß genug ist, um eine einfache Handhabung in einem durchschnittlichen Labor zu ermöglichen und zu akzeptablen Ergebnissen führt.

Zwar vertreibt Roche seit 2011 Heparin-beschichtete kapillare Blutentnahmesysteme (URL:https://www.roche.de/res/content/7687/blutentnahmesysteme bga.pdf, die jedoch nicht geeignet sind, hinreichend kleine Volumina zu transferieren und akzeptable Ergebnisse in der DNA-Amplifikation zu erzielen. Akzeptable Ergebnisse sind dabei klar erkennbare Amplifikatbanden in der DNA-Gelelektrophorese. Es hat sich gezeigt, dass ein PCR-Volumen von 10 µl diesbezüglich angemessen ist. US2002/012928 beschreibt die Verwendung von ein mit Heparin beschichtetes Reaktionsgefäss in Nukleinsäureamplifikationsverfahren.

### Zusammenfassung der Erfindung

Die der Erfindung zugrundeliegende Idee ist, in den Prozess der Probennahme und Transfer der Probe in die PCR eine Probenbehandlung einzubauen, der Art, dass während des Prozesses des Probentransfers das Probentransferwerkzeug Einfluss auf die Probe nimmt, d.h. dass das Werkzeug PCR-Inhibitoren durch z.B. Bindung von PCR-Inhibitoren oder durch Freisetzung von PCR-Inhibitor inaktivierenden Substanzen unschädlich macht oder zumindest die schädliche Wirkung reduziert, so dass besser oder überhaupt erst in der nachfolgenden PCR DNA amplifizieren werden kann. Es wurde festgestellt, dass der Transfer von sehr kleinen Blutmengen als Template in eine 10 µl umfassende PCR sehr einfach mittels eines mit Wirkstoff (z.B. Antikoagulantien) beschichteten Stäbchens geeigneter Länge, Form und Material durchgeführt werden kann. Die vorliegende Erfindung betrifft somit
(1) die Verwendung eines stabförmigen Probentransferwerkzeugs zum direkten Transfer von kleinen Nukleinsäure-haltigen Proben in eine enzymatische Reaktion, wobei das Probetransferwerkzeug einen Bereich aufweist, der zum Kontakt mit der Nukleinsäure-haltigen Probe bestimmt ist und der mit einem oder mehreren Wirkstoffen beschichtet ist, der/die in einer nach einem Transfer erfolgenden enzymatische Reaktion diejenigen in der Nukleinsäure-haltigen Probe enthaltenen Stoffe inhibiert(en), inaktiviert(en) oder deren inhibitorische Wirkung reduziert(en), die die nach dem Transfer erfolgende enzymatische Reaktion hemmen, in dem diese in der Probe enthaltene Stoffe zumindest teilweise gebunden oder inaktiviert werden;
(2) ein Nachweisverfahren umfassend:
   (a) in-Kontakt-bringen von einem stabförmigen Probentransferwerkzeug wie in Aspekt (1) definiert mit einer festen oder flüssigen Phase einer Nukleinsäure-haltigen Probe, so dass eine ausreichende Menge der Nukleinsäure-haltigen Probe an dem Probentransferwerkzeug haften bleibt,
   (b) Transfer des Probentransferwerkzeugs zu einem Reaktionsgemisch für eine enzymatische Nachweisreaktion und Eintauchen des Probentransferwerkzeugs in das Reaktionsgemisch, so dass eine für die Nachweisreaktion hinreichende Menge der Nukleinsäure-haltigen Probe in die Enzymreaktion eingetragen wird,
   (c) Entfernen des Probentransferwerkzeug aus dem Reaktionsgemisch für die enzymatische Nachweisreaktion; und
   (d) Durchführung der enzymatischen Nachweisreaktion; und
(3) die Verwendung eines Kits, umfassend ein oder mehrere stabförmige Probentransferwerkzeuge wie in Aspekt (1) definiert, zur Durchführung des Nachweisverfahrens von Aspekt (2).

### Kurzbeschreibung der Figuren

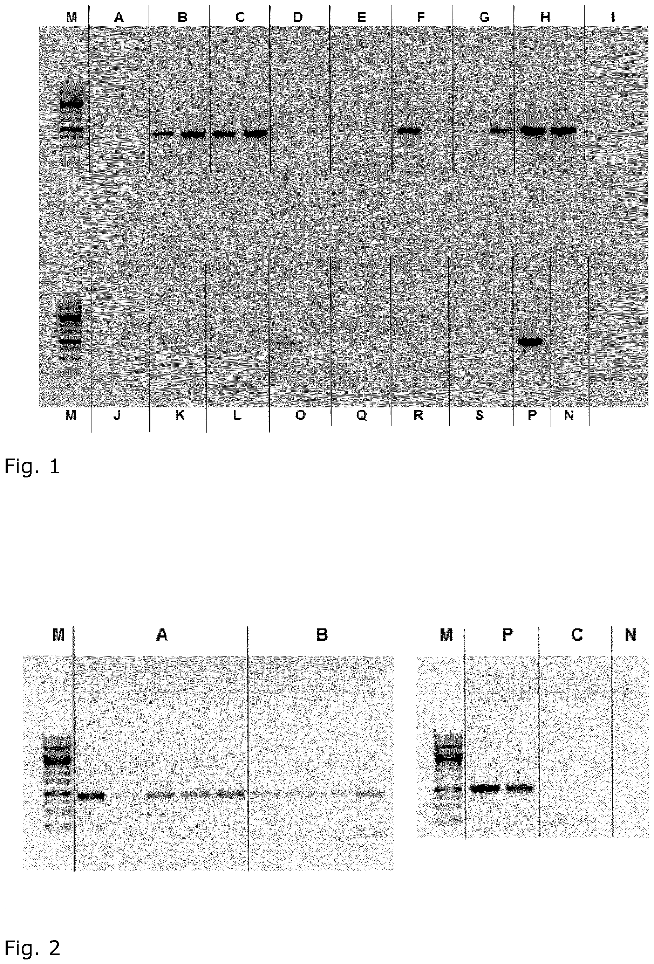

Die Figuren 1-43 zeigen die Ergebnisse der Beispiele 1-43.

### Detaillierte Beschreibung der Erfindung

Der Transfer von kleinen Probenmengen (z.B. Blut oder Bakterien aus einer Petrischale) mittels eines Stäbchens (z.B. Zahnstocher oder Pipettenspitze) ist schon seit langem bekannt und wird auch angewendet ("Colony PCR"). Die vorliegende Erfindung beruht auf dem überraschenden Befund, dass durch Vorbehandlung des Probentransferstäbchens mit einem geeigneten Wirkstoff, dieses mit einer Inhibitorinaktivierenden Funktion ausgestattet werden kann. Dem Fachmann ist hinlänglich bekannt, dass Heparin an und für sich eine stark PCR-inhibitorische Wirkung hat (vgl. EP-B-2373804, Seite 2 Abschnitt 7), insbesondere, wenn gereinigte DNA als Template in der PCR verwendet wird. Ebenfalls bekannt für den Fachmann, dass Polyanionen wie Heparin und andere sulfatisierte Polysaccharide als Inhibitoren der thermostabilen Polymerasen in PCR-Reaktionen eingesetzt werden können (vgl. US Patent 6,667,165). In EP-B-2373804 wurde jedoch weiterhin festgestellt, dass mit Heparin behandelte Blutproben und Verwendung dieser Blutproben als Template in direkt-PCR, weniger PCR-inhibitorische Wirkung aufweisen, als Blutproben welche mit Citrat oder EDTA antikoaguliert wurden. Offensichtlich "neutralisieren" sich PCR-inhibitorische Wirkungen von Heparin und PCR-Inhibitoren in Vollblut gegenseitig, so dass heparinisiertes Blut in der direkt-PCR wesentlich besser als Template für die PCR funktioniert, als EDTA- oder Citrat-behandeltes Blut. Überraschender Weise wurde festgestellt, dass mit geeigneten Wirkstoffen wie Heparin behandelte Stäbchen (Kunststoffzahnstocher) die technische Aufgabe lösen können. Dies mündete in der erfindungsgemäßen Verwendung eines Probentransferwerkzeugs gemäß Aspekten (1) bis (3) wie vorstehend beschrieben.

In dem Probentransferwerkzeug werden dabei bevorzugt solche Wirkstoffe eingesetzt, die Stoffe aus dem Probenmaterial, welche eine nach dem Transfer erfolgende enzymatische Nachweisreaktion hemmen, inaktivieren. Weiterhin ist es bevorzugt das die Wirkstoffe bei Kontakt mit der Probe in die Probe abgegeben oder in der Probe gelöst werden.

Die eingesetzten Wirkstoffe sind dabei solche Verbindungen, welche die nach dem Transfer erfolgende enzymatische Reaktion diejenigen in der Nukleinsäure-haltigen Probe enthaltenen Stoffe inhibieren, inaktivieren oder deren inhibitorische Wirkung reduzieren, die die nach dem Transfer erfolgende enzymatische Reaktion hemmen. Dabei kann das erfindungsgemäße Probentransferwerkzeug entweder durch die Wirkstoffe diese Verbindungen/Inhibitoren binden oder die Wirkstoffe abgeben, um dadurch die Inhibitoren zu inaktivieren. Das erfindungsgemäße Probentransferwerkzeug ist geeignet Probenmaterial in eine enzymatische Reaktion, insbesondere eine Nachweisreaktion, einzutragen. Die enzymatische Nachweisreaktion ist dabei bevorzugt ausgewählt aus PCR, Reverser Transkription, isothermaler Amplifikation, Restriktion, Sequenzierung und Nukleinsäureamplifikation, und die Wirkstoffe sind bevorzugt ausgewählt aus Antikoagulantien und polymeren Verbindungen wie Heparin, Chondrotinsulfat, Heparansulfat, Keratansulfat, Amidopektinen, Hyaluronsäure, Pektinen, Xanthan, Chitosan, Oligo-Glucosamin, Dextransulfat, Carrageen, Methylcellulose, Guaran und anderen Stoffen wie Chaotropsalze (z.B. Barium-, Calcium- und Guanidiniumperchlorate, -thiocyanate und -perchloracetate), für die bekannt ist, dass sie enzymatische Nachweisreaktionen hemmende/inhibitorische Stoffe inaktivieren. Dabei ist/sind der/die Wirkstoffe in einer ausreichenden Inhibitor-hemmenden Menge auf dem Probentransferwerkzeug vorhanden, wobei insbesondere für Heparin eine Beschichtung von 0,001 bis 0,5, bevorzugt 0,01 bis 0,1 IE/cm² Probentransferwerkzeugoberfläche, für andere Polysaccharide eine Beschichtung von 0,005 bis 500, bevorzugt 0,02 bis 25 µg/cm² Probentransferoberfläche und für Chaotropsalze eine Beschichtung von 0,1 bis 50, bevorzugt 1,5 bis 4 µmol/cm² Probentransferwerkzeugoberfläche auf dem Probentransferwerkzeug vorhanden ist.

Das Probentransferwerkzeug kann dabei die Form eines Stäbchens, eines Stiftes oder einer Nadel aufweisen und kann aus einem festen Material wie Kunststoff, Holz, Pappe, Papier, Keramik, Glas, Ton, Magnesia, Metall oder Kombinationen daraus bestehen. Das Probentransferwerkzeug weist einen Bereich auf, der zum Kontakt mit der Nukleinsäure-haltigen Probe bestimmt ist und der mit den Wirkstoffen beschichtet ist. Das Probenwerkzeug kann weiterhin einen Bereich aufweisen, mit dem es durch den Operator, einschließlich einem Menschen oder einer Maschine, gehalten wird.

Das Probentransferwerkzeug sollte dabei vorzugsweise so dimensioniert sein, dass es eine Probenmenge von 10 bis 500 nl, vorzugsweise von 50 bis 200 nl transferieren (bzw. 10 bis 500 µg, vorzugsweise 50 bis 200 µg, im Falle von Feststoffen) kann. Das Probentransferwerkzeug sollte vorzugsweise so mit dem/den Wirkstoff(en) beschichtet sein, dass eine ausreichende Inhibitor-hemmenden Menge des/der Wirkstoff(e) auf dem Probentransferwerkzeug vorhanden ist, z.B. Mengen wie sie vorstehend definiert sind. Das Probentransferwerkzeug kann neben den vorstehend genannten Wirkstoffen noch mit weitere neutralen Detergentien (wie z.B. Polyoxyethylenderivate von Sorbitanmonolaureat bzw. Sorbitanmonopalmitat der Tween^{®} Serie und Polyoxyethylenderivate von Fettalkoholen der Brij^{®} Serie) und mit Salzen (wie z.B. nicht-chaotrope Salze und Puffer) beschichtet sein.

Besonders bevorzugt ist dabei ein Probentransferwerkzeug, das aus einem Plastikstäbchen, Zahnstocher oder Kunststoffzahnstocher besteht und das mit Heparin beschichtet ist. Hierzu eignen sich z.B. Zahnstocher, bevorzugt Kunststoffzahnstocher einer speziellen Sorte. Nach dem In-Kontakt-bringen der Stäbchenspitze mit der Blutprobe haften verlässlich ca. 50 bis 200 nl Blut an deren Stäbchenspitze. Tippt man mit dieser Spitze in einen 10 µl PCR-Ansatz, wird die Blutmenge teilweise oder ganz in den PCR-Ansatz transferiert. Die durch das Stäbchen aufgenommene und abgegeben Blutmenge ist besser/genauer zu kontrollieren, als mit gebräuchlichen 0,5 bis 10 µl Pipettenspitzen oder Holzzahnstochern. Mittels dieser Methode lassen sich sehr gut PCRs in einem Volumen von 10 µl mit folgenden Blutproben durchführen: Human, Katze, Schaf, Meerschweinchen, Rind.

Die Herstellung eines solchen Probentransferwerkzeugs erfolgt durch Beladung mit Wirkstoffen entweder durch einfaches oder mehrfaches Tauchen des Ausgangs-Probentransferwerkzeugs (dem unbehandeltem Stäbchen) in eine wässrige Lösung mit einem oder mehreren Wirkstoffen und nachfolgendem Trocknen des Probentransferwerkzeugs, oder durch ein kovalentes Binden des Wirkstoffes oder der Wirkstoffe an das Ausgangs-Probentransferwerkzeug. Das Verfahren, das Tauchen/Trocknen umfasst, ist unter kommerziellen Gesichtspunkten sicherlich bevorzugt. Wie bereits vorstehend erwähnt, sind die Wirkstoffe vorzugsweise ausgewählt aus Antikoagulantien und polymeren Verbindungen wie Heparin, Chondrotinsulfat, Heparansulfat, Keratansulfat, Amidopektinen, Hyaluronsäure, Pektinen, Xanthan, Chitosan, Oligo-Glucosamin, Dextransulfat, Carrageen, Methylcellulose, Guaran und anderen Stoffen, für die bekannt ist, dass sie enzymatische Reaktionen hemmende/inhibitorische Stoffe inaktivieren.

Bei der Verwendung nach Aspekt (1) der Erfindung, ist die enzymatische Reaktion vorzugsweise eine enzymatische Nachweisreaktion, die insbesondere ausgewählt ist aus PCR, Reverser Transkription, isothermaler Amplifikation, Restriktion, Sequenzierung und Nukleinsäureamplifikation. Bei dieser Verwendung können die folgende Art von Proben untersucht werden, Körperbestandteile und Körperflüssigkeiten, einschließlich Blut, Urin, Speichel, Zellen, Gewebe und Stuhl, Pflanzen und Pflanzenbestandteilen, einschließlich jeglicher Art von Pflanzenmaterial, wobei Körperflüssigkeiten, Pflanzenmaterial und Stuhlproben besonders bevorzugt sind.

In dem erfindungsgemäßen Nachweisverfahren von Aspekt (2) nimmt das Probentransferwerkzeug auf die Nukleinsäure-haltigen Probe dahin gehend Einfluss, dass in der Probe enthaltene Stoffe, die die enzymatische Nachweisreaktion hemmen, mindestens teilweise inaktiviert oder gebunden werden. Es ist dabei bevorzugt, dass die enzymatische Nachweisreaktion ausgewählt ist aus PCR, Reverser Transkription, isothermaler Amplifikation, Restriktion, Sequenzierung und Nukleinsäureamplifikation. Wie vorstehend zu Aspekt (1) ausgeführt, können auch hier die Proben ausgewählt sein aus Körperbestandteilen und Körperflüssigkeiten, einschließlich Blut, Urin, Speichel, Zellen, Gewebe und Stuhl, Pflanzen und Pflanzenbestandteilen, einschließlich jeglicher Art von Pflanzenmaterial, wobei Körperflüssigkeiten, Pflanzenmaterial und Stuhlproben besonders bevorzugt sind. In beiden Aspekten (1) und (2) bedeutet "Transfer ... in ein Reaktionsgemisch", dass sich das Reaktionsgemisch in einem offenen oder verschließbaren Reaktionsgefäß, in einem Napf einer (Mikro-)Titerplatte oder auf einer ebenen Platte befinden kann.

In dem erfindungsgemäßen Nachweisverfahren können die Schritte (a) und (b), unabhängig voneinander, manuell durch einen Operator, oder maschinell durchgeführt werden. Hierbei ist es bevorzugt, dass durch die Schritte (a) und (b) eine Probenmenge von 10 bis 500 nl, vorzugsweise von 50 bis 200 nl transferiert wird (bzw. 10 bis 500 µg, vorzugsweise 50 bis 200 µg bei Feststoffen). Weiterhin ist es bevorzugt, dass das Reaktionsvolumen der Nachweisreaktion 1 bis 100 µl, vorzugsweise etwa 10 µl beträgt. Optional kann in dem erfindungsgemäßen Nachweisverfahren vor dem in-Kontakt-bringen von Schritt (a) eine Lyse des Probenmaterials erfolgen.

Der in der Verwendung nach Aspekt (3) der Erfindung eingesetzte Kit kann weiterhin Reagenzien, Reaktionsgemische und/oder Lösungsmittel für die Nachweisreaktion und/oder Reagenzien für die Lyse des Probenmaterials enthalten.

Die Erfindung wird anhand der nachfolgenden Beispiele näher erläutert

### Beispiele

### Materialien und Methoden

Übergreifend für alle Versuche, sofern nicht anders angegeben:
Herstellung Probentransferwerkzeug: Ein Stäbchen, z.B. ein handelsüblicher Kunststoffzahnstocher wird für einige Sekunden bis einige Minuten in eine Lösung eingetaucht, die besteht aus wässriger Lösung enthaltend: Als Wirkstoff: 38 IE Heparin je ml oder einen anderen Wirkstoff wie in den Beispielen angegeben; als Hilfstoff: 10% Polysorbat 20 (CAS-Nr. 9005-64-5, Handelsname z.B. Tween^{®} 20) oder Polypropylenglykol (CAS 25322-69-4, durchschnittliches Molekulargewicht: 2700 g/mol). Nach Trocknung für mindestens eine Stunde wird das Werkzeug zum Probentransfer verwendet.
PCR-Mix: MACHEREY-NAGEL NucleoType HotStart PCR Master Mix enthaltend Hotstart Taq DNA-Polymerase, Puffer, Magnesiumionen, dNTPs, roter Farbstoff. Reaktionsansatz von 10 µl enthält: 5 µl NucleoType HotStart PCR Master Mix, je 0,2 µM eines jeden Primer, Wasser. In diesen Reaktionsansatz wird mittels Transfer Tool Probe hinzugegeben. Typischer Reaktionsansatz für die PCR für eine Probe: 5 µl NucleoType HotStart PCR Master Mix; 0,2 µl Primer A (10 µM); 0,2 µl Primer B (10 µM); 4,6 µl Wasser; 10 µl Gesamtvolumen.
Probentransfer: Das Werkzeug wird kurz (ca. eine Sekunde) um ca. 1 - 5 mm tief in die Probe eingestochen und hiernach kurz (ca. eine Sekunde) in den PCR eingedippt, ohne weitere Misch- oder Rührbewegungen auszuführen.
PCR-Gerät: Als PCR-Gerät wurde entweder ein MasterCycler Gradient (Firma Eppendorf) oder ein Thermocycler GeneExplorer Triple oder Thermocycler GeneExplorer Advanced (Firma JoJo Life Sciences) verwendet.
Analytik der PCR-Produkte: Nach Durchführung der PCR wurden die Reaktionsansätze mittels Gelelektrophorese (1% Agarosegel in TAE-Puffer, Anfärbung mittels Ethidiumbormid) oder mittels *Agilent 2100 Bioanalyzer* und *Agilent DNA 1000 Kit* laut Herstellerangaben analysiert.

**Tabelle 1: Verwendete Primer**

| **Primerpaar #** | **SEQ ID NO:** | **Sequenz 5' - 3'** | **Amplifikatgröße (bp)** | **Ziel bereich** |
|---|---|---|---|---|
| | | | | **Mt: Mitochondrial** |
| | | | | **Kk: Kernkodiert** |
| | | | | **Spü: Speziesübergreifend** |
| | | | | **Spsp: Speziesspezifisch** |
| 1 | 1 | F: GGAATGGCATTGACTTGGTC | 813 | Bitter-Taste Receptor Gene Repertoire in Different Lagomorphs Species |
| | 2 | R: GTTCTCGGAACCGATCACA | | |
| 2 | 3 | F: CTGAAGCTTTTGGCTTTGAG | female: 331 bp | Kk, sex determination |
| | 4 | R: CCGCTGCCAAATTCTTTGG | | |
| | | | male: 302 + 331 bp | |
| 3 | 5 | F: AACGACCCCTTCATTGAC | 191 | Kk, GAPDH, spü, im Mausgenom mehrere dutzend bis zu ca. 200 Targets |
| | 6 | R: TCCACGACATACTCAGCAC | | |
| 4 | 7 | F: CTCCTACCTCTACAA | 415 | Spsp, Huhn |
| | 8 | R: GGCTAGTGTTAGGAAT | | |
| 5 | 9 | F: GAGATGTTTTTGCTGGTATTGA | 308 | ManyPlants |
| | 10 | R: AAYGTATAAACCAATGCTTCCAT | | |
| 6 | 11 | F: GACGGGAATTGAACCCGCG | 447 | ManyPlants |
| | 12 | R: GTTATGCATGAACGTAATGCTC | | |
| 7 | 13 | F: GCCCTCTACTCCACCCCCATCC | 118 | Spsp, Soja |
| | 14 | R: GCCCATCTGCAAGCCTTTTTGTG | | |
| 8 | 15 | F: CCGCTGTATCACAAGGGCTGGTACC | 226 | Spsp, Mais |
| | 16 | R: GGAGCCCGTGTAGAGCATGACGATC | | |
| 9 | 17 | F: GTAACTTCCAAATTCAGAGAAAC | 201 | Spsp, Weizen |
| | 18 | R: TCTCTAATTTAGAATTAGAAGGAA | | |
| 10 | 19 | F: CACAGTATTTGCTCGCTGAGA | 422 | Spsp, Tabak |
| | 20 | R: CCATTGCTGCTTCTTCTCC | | |
| 11 | 21 | F: TTAATTCAAAAATCATTTTTCCCG | 230 | Spsp, Arabidopsis |
| | 22 | R: TCAATGAAAGTCCCATTCTTTG | | |
| 12 | 23 | F: TTCCGAACCGAATCAAGG | 193 | Vitis vinifera |
| | 24 | R: GGAGCACCGTTCCAAGC | | |
| 13 | 25 | F: CAWCGATGAAGAACGYAGC | 418 | Spü, Pflanzen, Pilze |
| | 26 | R: RGTTTCTTTTCCTCCGCTTA | | |
| 14 | 27 | F: GGAAGKARAAGTCGTAACAAGG | 745 | Spü, Pflanzen, Pilze |
| | 28 | R: RGTTTCTTTTCCTCCGCTTA | | |
| duplex 15 | 29 | F: CCTTATCAYTTAGAGGAAGGAG | 757 | Spü, Pflanzen, Pilze |
| | 30 | R: RGTTTCTTTTCCTCCGCTTA | | |
| (13) | 25 | F: CAWCGATGAAGAACGYAGC | 418 | |
| | 26 | R: RGTTTCTTTTCCTCCGCTTA | | |
| 16 | 31 | F: CTAGAGGAGCCTGTTCTATAATCGATAA | 142 | Spsp, Ziege |
| | 32 | R: TGACCTAACGTCTTTATGTGTGGTG | | |

**Tabelle 2: Verwendete PCR-Programme, -Geräte**

| **PCR-Programm #** | **Programm** | **Gerät** |
|---|---|---|
| 1 | 1x: 95°C 2 min | Eppendorf Gradient Cycler |
| | 40x: 95°C, 15 s; 55°C, 15 s; 72°C, 15 s | |
| | 1x: 72°C, 1 min | |
| | Kühlung | |
| 2 | 1x: 95°C 2 min | Eppendorf Gradient Cycler |
| | 40x: 95°C, 15 s; 60°C, 15 s; 72°C, 15 s | |
| | 1x: 72°C, 1 min | |
| | Kühlung | |
| 3 | 1x: 95°C 2 min | Eppendorf Gradient Cycler |
| | 40x: 95°C, 15 s; 38°C, 15 s; 72°C, 15 s | |
| | 1x: 72°C, 1 min | |
| | Kühlung | |
| 4 | 1x: 95°C 2 min | Eppendorf Gradient Cycler |
| | 40x: 95°C, 15 s; 65°C, 15 s; 72°C, 15 s | |
| | 1x: 72°C, 1 min | |
| | Kühlung | |
| 5 | 1x: 95°C 2 min | Eppendorf Gradient Cycler |
| | 40x: 95°C, 15 s; 52°C, 15 s; 72°C, 15 s | |
| | 1x: 72°C, 1 min | |
| | Kühlung | |
| 6 | 1x: 95°C 2 min | Thermocycler GeneExplorer Advanced |
| | 40x: 95°C, 15 s; 60°C, 20 s; 72°C, 30 s | |
| | 1x: 72°C, 1 min | |
| | Kühlung | |
| 7 | 1x: 95°C 2 min | Thermocycler GeneExplorer Advanced |
| | 40x: 95°C, 15 s; 55°C, 30 s; 72°C, 1 min | |
| | 1x: 72°C, 1 min | |
| | Kühlung | |
| 8 | 1x: 95°C 2 min | Thermocycler GeneExplorer Advanced |
| | 40x: 95°C, 15 s; 60°C, 30 s; 72°C, 1 min | |
| | 1x: 72°C, 1 min | |
| | Kühlung | |
| 9 | 1x: 95°C 2 min | Thermocycler GeneExplorer Advanced |
| | 40x: 95°C, 15 s; 55°C, 20 s; 72°C, 30 s | |
| | 1x: 72°C, 1 min | |
| | Kühlung | |
| 10 | 1x: 95°C 2 min | Thermocycler GeneExplorer Advanced |
| | 40x: 95°C, 15 s; 65°C, 20 s; 72°C, 30 s | |
| | 1x: 72°C, 1 min | |
| | Kühlung | |
| 11 | 1x: 95°C 2 min | Thermocycler GeneExplorer Advanced |
| | 40x: 95°C, 15 s; 52°C, 30 s; 72°C, 1 min | |
| | 1x: 72°C, 1 min | |
| | Kühlung | |
| 12 | 1x: 95°C 2 min | GET3X Thermal Cycler |
| | 40x: 95°C, 15 s; 55°C, 20 s; 72°C, 30 s | |
| | 1x: 72°C, 1 min | |
| | Kühlung | |
| 13 | 1x: 98°C 5 min | Eppendorf Gradient Cycler |
| | 40x: 98°C, 5 s; 55°C, 5 s; 72°C, 20 s | |
| | 1x: 72°C, 1 min | |
| | Kühlung | |
| 14 | 1x: 94°C 3 min | Eppendorf Gradient Cycler |
| | 30x: 94°C, 30 s; 55°C, 30 s; 72°C, 20 s | |
| | 1x: 72°C, 10 min | |
| | Kühlung | |
| 15 | 1x: 95°C 3 min | Eppendorf Gradient Cycler |
| | 30x: 95°C, 15 s; 55°C, 15 s; 72°C, 45 s | |
| | Kühlung | |
| 16 | 1x: 95°C 3 min | Eppendorf Gradient Cycler |
| | 35x: 95°C, 20 s; 55°C, 30 s; 72°C, 30 s | |
| | 1x: 72°C, 1 min | |
| | Kühlung | |
| 17 | 1x: 95°C 2 min | GET3X Thermal Cycler |
| | 40x: 95°C, 15 s; 60°C, 20 s; 72°C, 30 s | |
| | 1x: 72°C, 1 min | |
| | Kühlung | |
| 18 | 1x: 95°C 2 min | GET3X Thermal Cycler |
| | 40x: 95°C, 15 s; 60°C, 30 s; 72°C, 30 s | |
| | 1x: 72°C, 1 min | |
| | Kühlung | |

### Übersicht

Beispielgruppe I: Blutproben (mit Heparin-behandelte Probentransferwerkzeuge).
   Beispiel 1: Verschiedene Heparinkonzentrationen in Wasser und Tween^{®} 20
   Beispiel 2: Verschiedene Eintauchtiefen
   Beispiel 3: Tween^{®} 10%ig und Brij 1%ig als Hilfsstoffe
   Beispiel 4: 20 Replikate. Kontrolle: Tween^{®} ohne Heparin
   Beispiel 5: 20 Replikate. Kontrolle: Probentransferwerkzeug unbehandelt
   Beispiel 6: Eintauchtiefen in Heparin und Blut
   Beispiel 7: Robustheit Werkzeug: mechanische Einwirkung und anderes
   Beispiel 8: Diverse Blutproben
Beispielgruppe II: Pflanzenblattproben (mit Heparin-behandelte Probentransferwerkzeuge)
   Beispiel 9: Weizen, Mais, Baumwolle: Werkzeug behandelt vs. unbehandelt
   Beispiel 10: Soja: Werkzeug behandelt vs. unbehandelt
   Beispiel 11: Soja, Weizen, Mais, Tabak: Werkzeug behandelt. Einfache Kontrollen
   Beispiel 12: Arabidopsis, Probennahme: durchstechen, kratzen
   Beispiel 13: Tabak, Probennahme: anstechen, kratzen. Dauer Probentransfer 0-4 s
   Beispiel 14: Tabak, Probennahme und -transfer: stechen, kratzen, 1x, 2x antippen, einrühren
   Beispiel 15: Mechanische Stabilität der Beschichtung. Tabak
   Beispiel 16: Duplex PCR Tabak-Wein, Bioanaylzer
   Beispiel 17: Soja, Baumwolle, Weizen, Amplikon 418 bp
   Beispiel 18: Baumwolle, Weizen, Amplikon 745 bp
   Beispiel 19: Tabak, Weinblätter: Nach Probenaufnahme direkter Probentransfer vs. Eintrocknung Probe
   Beispiel 20: Duplex PCR Soja, Baumwolle, Weizen, 418 bp + 757 bp
   Beispiel 21: Probennahme: Einstechen vs. Durchstechen, Tabak, Wein
   Beispiel 22: Beschichtungsprozedur: Einzeln oder im Bündel.
   Beispiel 23: Vergleich: Lysat vs. direkter Probentransfer. Tabak, Baumwolle
   Beispiel 24: PCR-Volumen 10 - 50 µl, Tabak, Baumwolle
   Beispiel 25: Lagerung der Beschichtung: frisch vs. 2 Monate bei -20 °C bis +37 °C.
   Beispiel 26: Produktvergleich
Beispielgruppe III: Andere Probenarten
   Beispiel 27: Avocadofruchtfleisch: Werkzeug +/- Heparin
   Beispiel 28: Avocadofruchtfleisch: Werkzeug +/- Heparin
   Beispielgruppe IV: Unterschiedliche Wirkstoffe
   Beispiel 29: Chondrotinsulfat 5%, 1% im Vergleich zu Heparin und Negativkontrolle
   Beispiel 30: Dextransulfat 0,1 - 5% im Vergleich zur Heparinkontrolle und NK, Maus EDTA-Blut
   Beispiel 31: Chaotropsalzlösung anstelle von Heparin
   Beispiel 32: Carrageenan 0,05 - 0,5%, Kaninchenblut
   Beispiel 33: Dextransulfat 0,01% - 1,5% mit Tabak
   Beispiel 34: Arabidopsis: Heparin, Dextran vs. Unbeschichtet und NK
   Beispiel 35: Mais: Dextran 0,001%, 0,005%, 0,01% vs. unbeschichtet
   Beispiel 36: GITC und GuHCl +/- Tween^{®} mit Weinblatt
   Beispiel 37: Tween^{®} als Hilfsstoff ersetzt durch Wasser bzw. Propylenglykol mit Blätter und Blut
   Beispiel 38: λ-Carrageenan mit Blut
   Beispiel 39: λ-Carrageenan mit Fleisch (Ziege)
Beispielgruppe V: Unterschiedliche Materialien und Formen des Probentransferwerkzeugs
   Beispiel 40: Zahnstocher, Pipettenspitzen, Büroklammer
   Beispiel 41: Combitips Innenteil, Büroklammer mit Baumwollblatt
   Beispiel 42: Polystyrolstäbchen aus Wägeschiffchen mit Blut
   Beispiel 43: Pipettenspitze mit Blut

### Beispiel 1: Verschiedene Heparinkonzentrationen in Wasser und Tween^{®} 20 Probenmaterial: EDTA-Kaninchenblut

Werkzeugbeschreibung (Art und Menge von Wirk- und Hilfsstoffen): Kunststoffzahnstocher beschichtet mit Li-Heparin +/- Tween^{®} 20 verschiedener Mengen.

Beschichtungslösungen:
Heparinmenge in Internationalen Einheiten (IE) je 500 µl
Wasser: 120 IE; 12 IE ÜW (vom Vortag); 1,2 IE fr. (frisch hergestellt); 0,12 IE. 0,1% Tween^{®} 20: 120 IE; 12 IE; 1,2 IE; 0,12 IE, 0 IE.
P: Positivkontrolle: 50 µl Blut mit 1,5 IE Heparin behandelt, Zahnstocher unbehandelt
N: Blut unbehandelt, Probentransferwerkzeug unbehandelt
Primer: Primerpaar # 1
PCR-Programm: # 2
Das Ergebnis ist in Figur 1 gezeigt (Heparinmenge in Internationalen Einheiten (IE) je 500 µl): M - Marker; A - 120 IE Heparin in Wasser; B - 12 IE Heparin in Wasser, vom Vortag; C - 12 IE Heparin in Wasser, frisch hergestellt; D - 1,2 IE Heparin in Wasser; E - 0,12 IE Heparin in Wasser; F - 0 IE Heparin in Wasser; G - 120 IE Heparin in 0,1% Tween^{®} 20; H - 12 IE Heparin in 0,1% Tween^{®} 20; I - 1,2 IE Heparin in 0,1% Tween^{®} 20; J - 0,12 IE Heparin in 0,1% Tween^{®} 20; K - 0 IE Heparin in 0,1% Tween^{®} 20; L - 120 IE Heparin in 1% Saccharose; O - 12 IE Heparin in 1 % Saccharose; Q - 1,2 IE Heparin in 1% Saccharose; R - 0,12 IE Heparin in 1 % Saccharose; S - 0 IE Heparin in 1% Saccharose; P - Positivkontrolle: 50 µl Blut mit 1,5 IE Heparin behandelt, Zahnstocher unbehandelt; N - Blut unbehandelt, Zahnstocher unbehandelt
Fazit: Bei Verwendung eines Probentransferwerkzeugs, welches mit einer Lösung aus Heparin in geeigneter Konzentration in Wasser oder in 0,1% Tween^{®} 20 beschichtet wurde, bildet sich in der PCR ein Amplifikat, wohingegen es bei Verwendung eines unbehandelten Zahnstochers zum Probentransfer nicht zur Amplifikation kommt (N). Bei der Positivkontrolle (P) wurde das Blut mit Heparin behandelt, bevor es mit einem unbeschichteten Zahnstocher in die PCR transferiert wurde.

### Beispiel 2: Verschiedene Eintauchtiefen:

### Probenmaterial: EDTA-Kaninchenblut

Werkzeugbeschreibung (Art und Menge von Wirk- und Hilfsstoffen): Kunststoffzahnstocher beschichtet mit Li-Heparin/ Tween^{®} 20. Zahnstocher unterschiedlich tief in Lösung zur Beschichtung eingetaucht.

Beschichtungslösungen:
120 IE Heparin in 500 µl 0,1 % Tween^{®}, Zahnstocher eingetaucht 10 mm oder 2 mm in Beschichtungslösung, P: Positivkontrolle: 50 µl Blut mit 1,5 IE Heparin behandelt, Zahnstocher unbehandelt, Ohne Heparin: Stäbchen unbeschichtet, N: PCR ohne Probenzugabe
Primer: Primerpaar # 1
PCR-Programm: # 2
Das Ergebnis ist in Figur 2 gezeigt: M - Marker; A - 120 IE Heparin in 500 µl 0,1 % Tween^{®}, Zahnstocher 10 mm in Beschichtungslösung eingetaucht; B - 120 IE Heparin in 500 µl 0,1 % Tween^{®}, Zahnstocher 2 mm in Beschichtungslösung eingetaucht; C - Ohne Heparin: Zahnstocher unbeschichtet; P - Positivkontrolle: 50 µl Blut mit 1,5 IE Heparin behandelt, Zahnstocher unbehandelt; N - Negativkontrolle: PCR ohne Templatezugabe
Fazit: Bei Verwendung eines Probentransferwerkzeugs, welches zur Beschichtung 2 mm oder 10 mm tief in die Beschichtungslösung eingetaucht wurde, ergeben sich PCR-Amplifikate, wohingegen sich bei Probentransfer mittels unbeschichteter Zahnstocher keine PCR-Amplifikate bilden.

### Beispiel 3: Tween^{®} 10 %ig und Brij^{®} 1 %ig als Hilfsstoffe

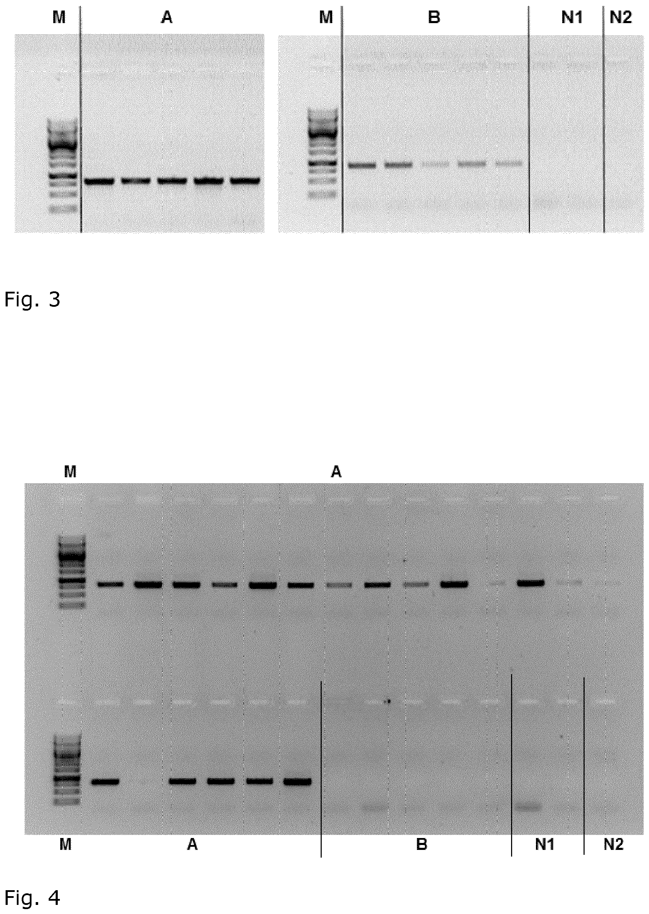

Probenmaterial: EDTA-Kaninchenblut
Werkzeugbeschreibung (Art und Menge von Wirk- und Hilfsstoffen): Kunststoffzahnstocher beschichtet mit folgenden Lösungen: 20 IE Heparin in 500 µl Lösung 10% Tween^{®} 20 oder 1% Brij^{®} 58 (CAS 9004-95-9)
Negativkontrolle "ohne Heparin" = Zahnstocher unbehandelt
N: Negativkontrolle, PCR ohne Template
Primer: Primerpaar # 1
PCR-Programm: # 2
Das Ergebnis ist in Figur 3 gezeigt: M - Marker; A - 20 IE Heparin in 500 µl Lösung 10% Tween^{®}20; B - 20 IE Heparin in 500 µl Lösung 1% Brij^{®} 58; N1 - Negativkontrolle "ohne Heparin", Zahnstocher unbehandelt; N2 - Negativkontrolle, PCR ohne Template
Fazit: Bei Verwendung eines Zahnstochers, der mit Heparin gelöst in 10% Tween^{®}20 oder in 1% Brij^{®} 58 beschichtet wurde, bilden sich Zielamplifikate, wohingegen ein Probentransfer mit unbeschichtetem Zahnstocher keine Amplifikate bildet. Als Hilfsstoff kann somit nicht nur das Detergenz Tween^{®} 20 sondern auch das Detergenz Brij^{®} 58 verwendet werden.

### Beispiel 4: 20 Replikate. Kontrolle: Tween ohne Heparin

Probenmaterial: EDTA-Kaninchenblut
Werkzeugbeschreibung (Art und Menge von Wirk- und Hilfsstoffen): Kunststoffzahnstocher beschichtet mit A: 20 IE Heparin/500 µl 10 %iges Tween^{®}20 (20 Replikate); B: 10 %iges Tween^{®}20 (5 Replikate);
Probentransfer: Zur Probenaufnahme wurden die Zahnstocher in 3 µl Blutaliquots eingetaucht.
Primer: Primerpaar # 1
PCR-Programm: # 2
Das Ergebnis ist in Figur 4 gezeigt: M - Marker; A - 20 IE Heparin / 500 µl 10 %iges Tween^{®}20 (20 Replikate); B - 10 %iges Tween^{®}20 ohne Heparin (5 Replikate); N1 - Negativkontrolle: Unbehandelte Zahnstocher; N2 - Negativkontrolle: PCR ohne Templatezugabe
Fazit: Bei Verwendung von Zahnstochern zum Probentransfer, welche nur mit Tween20-Lösung beschichtet wurde liefern 5 von 5 Reaktionen keine Amplifikate, wohingegen bei Verwendung von Zahnstochern, die mit Heparin/Tween^{®}-Lösung beschichtet wurden, 19 von 20 Rekationen Amplifikaten bilden. Tween^{®}20 stellt somit einen Hilfsstoff (Detergenz, Netzmittel) dar, keinen Wirkstoff.

### Beispiel 5: 20 Replikate. Kontrolle: Probentransferwerkzeug unbehandelt

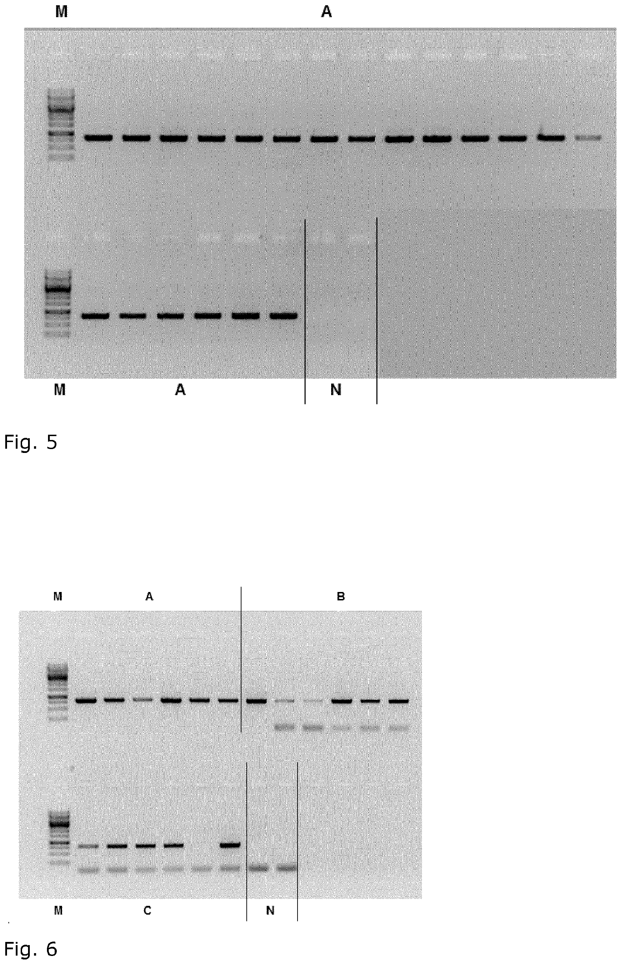

Probenmaterial: EDTA-Kaninchenblut
Werkzeugbeschreibung (Art und Menge von Wirk- und Hilfsstoffen): 20 IE Heparin/500 µl 10 %iges Tween^{®}20 (20 Replikate)
Probentransfer: Zur Probenaufnahme wurden die Zahnstocher in 10 µL Blutaliquots eingetaucht
Primer: Primerpaar # 1
PCR-Programm: # 2
Das Ergebnis ist in Figur 5 gezeigt: M - Marker; A - 20 IE Heparin / 500 µl 10 %iges Tween^{®}20 (20 Replikate); N - Negativkontrolle: Unbehandelte Zahnstocher
Fazit: Bei Verwendung von Zahnstochern zum Probentransfer, welche Heparin/Tween^{®}-Lösung beschichtet wurden und zur Probenaufnahme in 10 µl Blutaliquots tief eingetaucht wurden, liefern 20 von 20 Reaktionen Amplifikate, wohingegen bei Verwendung von unbehandelten Zahnstochern, 2 von 2 Rekationen keine Amplifikate bilden.

### Beispiel 6: Eintauchtiefen in Heparin und Blut

Probenmaterial: EDTA-Kaninchenblut
Werkzeugbeschreibung (Art und Menge von Wirk- und Hilfsstoffen): Kunststoffzahnstocher. Beschichtungslösung: 38 IE Heparin/ml Lösung enthaltend 10 % Tween^{®}20. Verschiedene Eintauchtiefen der Zahnstocher in die Beschichtungslösung: 9 mm, 5 mm, 13 mm. Verschiedene Eintauchtiefen des beschichteten Zahnstochers ins Blut: 5 mm, 13 mm. Eintauchtiefe des mit Blut beladenen Zahnstochers in die PCR: ca. 3 mm für alle.
Primer: Primerpaar # 1
PCR-Programm: # 2
Das Ergebnis ist in Figur 6 gezeigt: Eintauchtiefen des Zahnstochers; M: Marker, DNA-Leiter; A: Heparinlösung 10 mm, Blut 5 mm; B: Heparinlösung 5 mm, Blut 13 mm; C: Heparinlösung 13 mm, Blut 13 mm; N: unbehandelte Zahnstocher, Blut 5 mm
Fazit: Bei Verwendung von mit Heparinlösung behandelter Zahnstocher zum Probentransfer von Blut in die PCR lassen sich mit verschiedenen Eintauchtiefen der Zahnstocher in die Heparinlösung zu Beschichtung als auch mit verschiedenen Eintauchtiefen in die PCR zu Abgabe des Blutes Amplifikate bilden, wohingegen sich beim Bluttransfer mit unbeschichteten Zahnstochern keine Amplifikate bilden.

### Beispiel 7: Robustheit Werkzeug: mechanische Einwirkung und anderes

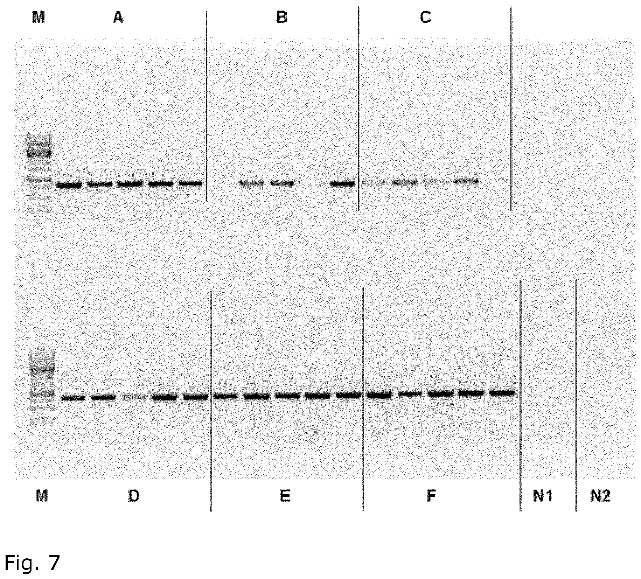

Probenmaterial: EDTA-Kaninchenblut
Werkzeugbeschreibung (Art und Menge von Wirk- und Hilfsstoffen): Kunststoffzahnstocher, Beschichtungslösung: 38 IE Heparin/ml Lösung enthaltend 10 % Tween20.
Primer: Primerpaar # 1
PCR-Programm: # 2
Das Ergebnis ist in Figur 7 gezeigt: A: Frisch hergestellte Probentransferwerkzeuge, Kontakt des blutbeladenen Werkzeugs mit der PCR für eine Sekunde; B: dto, aber Kontakt des blutbeladenen Werkzeugs mit der PCR für fünf Sekunden; C: dto, aber Kontakt des blutbeladenen Werkzeugs mit der PCR mit kreisender Bewegung in der PCR für ca. drei Sekunden; D: Wie A), jedoch Probentransferwerkzeug vor Blutaufnahme / Verwendung auf den Tisch falle lassen, um die mechanische Stabilität der Beschichtung zu testen; E: Wie A), aber Probentransferwerkzeuge verwendet, die ca. fünf Wochen alt waren; F: Wie D), aber Probentransferwerkzeuge verwendet, die ca. fünf Wochen alt waren; N1: Unbeschichtete Werkzeuge zum Bluttransfer verwendet; N2: PCR-Negativkontrolle (No Template Control)
Fazit: Mit Heparin beschichtete Zahnstocher sind für mindestens mehrere Wochen lagerstabil, überstehen eine mechanische Belastung (Hinfallen). Das Einbringen der Blutprobe in die PCR kann durch kurzzeitigen Kontakt (ca. eine Sekunde) aber auch - wenngleich mit etwas geringerem Erfolg - durch längeren Kontakt oder durch Einrühren erfolgen.

### Beispiel 8: Diverse Blutproben

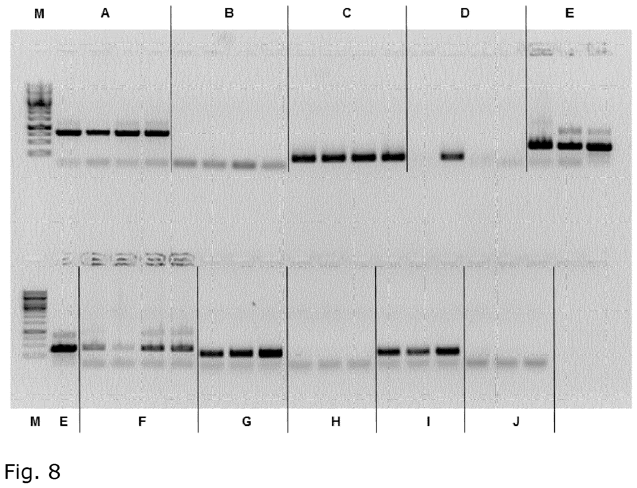

Probenmaterial: EDTA-Kaninchenblut, EDTA-Mausblut, Citrat-Mausblut, EDTA-Rattenblut, EDTA-Hühnerblut
Werkzeugbeschreibung (Art und Menge von Wirk- und Hilfsstoffen): Kunststoffzahnstocher, 38 IE Heparin/ml 10 %iger Tween^{®}20 Lösung
Primer: Primerpaar # 1 (Kaninchen), Primerpaar # 2 (Maus), Primerpaar # 3 (Ratte), Primerpaar # 4 (Huhn)
PCR-Programm: # 2 Kaninchen, Maus, Ratte bzw. # 3 für Huhn
Das Ergebnis ist in Figur 8 gezeigt: M: Marker - DNA-Leiter; A: EDTA-Kaninchenblut, Heparin beschichteter Zahnstocher; B: EDTA-Kaninchenblut, unbeschichteter Zahnstocher; C: EDTA-Rattenblut, Heparin beschichteter Zahnstocher; D: EDTA-Rattenblut, unbeschichteter Zahnstocher; E: EDTA-Hühnerblut, Heparin beschichteter Zahnstocher; F: EDTA-Hühnerblut, unbeschichteter Zahnstocher; G: EDTA-Mausblut, Heparin beschichteter Zahnstocher; H: EDTA-Mausblut, unbeschichteter Zahnstocher; I: Citrat-Mausblut, Heparin beschichteter Zahnstocher; J: Citrat-Mausblut, unbeschichteter Zahnstocher
Fazit: Die Verwendung von Zahnstochern zum Transfer von Blutprobenaliquots in die PCR ermöglicht oder verbessert die Bildung von Amplifikaten in der PCR, sofern sie mit Heparin beschichtet sind, im Vergleich zu unbeschichteten Zahnstochern. Dies gilt für Blutproben verschiedener Organismen und Antikoagulantien.

### Versuchsgruppe II: Pflanzenblattproben

### Beispiel 9: Weizen, Mais, Baumwolle: Werkzeug behandelt vs. unbehandelt

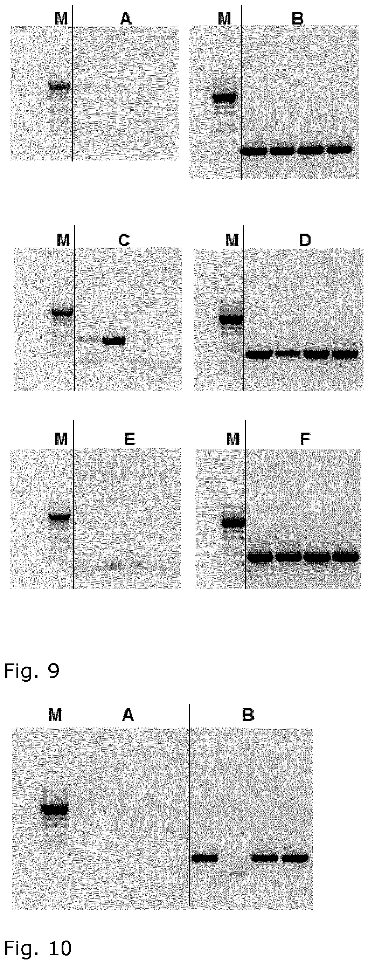

Probenmaterial: Blattmaterial von Weizen, Mais, Baumwolle
Werkzeugbeschreibung (Art und Menge von Wirk- und Hilfsstoffen): Kunststoffzahnstocher behandelt mit 38 IE Heparin/ml 10%iger Tween^{®}20 Lösung.
Primer: Primerpaar # 5 (Baumwolle), Primerpaar # 6 (Weizen und Mais)
PCR-Programm: # 1
Das Ergebnis ist in Fig. 9 gezeigt: M - Marker; A - Baumwolle, Werkzeug unbeschichtet; B - Baumwolle, Heparinwerkzeug; C - Weizen, Werkzeug unbeschichtet; D - Weizen, Heparinwerkzeug; E - Mais, Werkzeug unbeschichtet; F - Mais, Heparinwerkzeug
Fazit: Die Verwendung heparinbeschichteter Zahnstocher zum Transfer von Probenmaterial von Weizen-, Mais- und Baumwollblättern führt zur Bildung von erwarteten Amplifikationsprodukten. Bei Weizen und Mais wurde ein 447 bp Amplifikat, bei Baumwolle ein 308 bp Amplifikat gebildet.

### Beispiel 10: Soja: Werkzeug behandelt vs. unbehandelt

Probenmaterial: Pflanzenblattmaterial von Soja
Werkzeugbeschreibung (Art und Menge von Wirk- und Hilfsstoffen): 40 IE Heparin/ml 10 %igem Tween^{®}20
Primer: Primerpaar # 6
PCR-Programm: # 2
Das Ergebnis ist in Figur 10 gezeigt: M: Marker - DNA-Leiter; A: Unbehandelter Zahnstocher zum Transfer von Sojabohnenblattmaterial in die PCR verwendet. Vier Proben; B: Mit Heparin/Tween^{®} beschichteter Zahnstocher zum Transfer von Sojabohnenblattmaterial in die PCR verwendet. Vier Proben
Fazit: Durch Verwendung heparinbehandelter Zahnstocher zum Transfer von Sojabohnenblattmaterial in die PCR verbessert sich die Amplifikatbildung im Vergleich zur Verwendung unbehandelter Zahnstocher.

### Beispiel 11: Soja, Weizen, Mais, Tabak: Werkzeug behandelt. Einfache Kontrollen

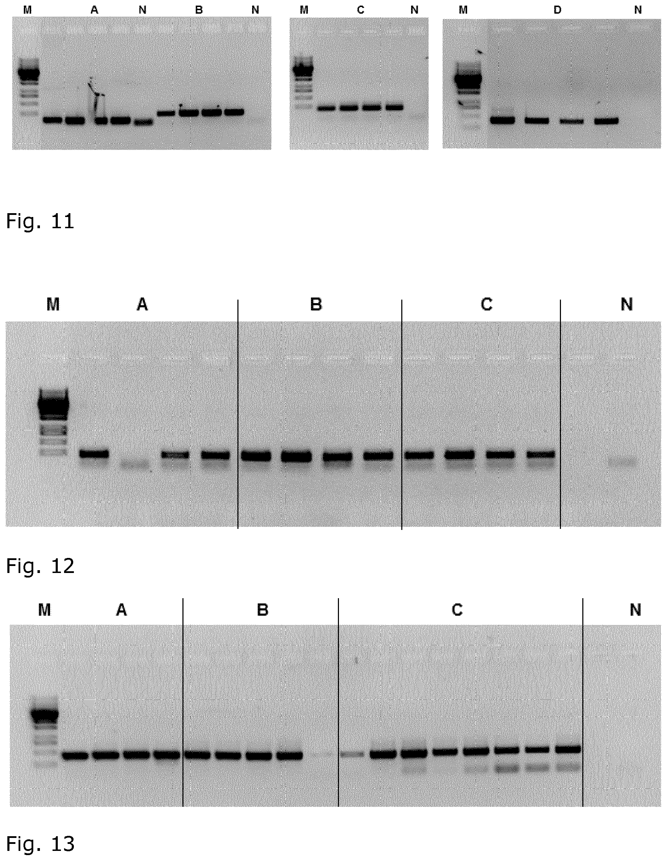

Probenmaterial: Blätter von Arabidopsis, Weizen, Soja, Mais, Tabak
Werkzeugbeschreibung (Art und Menge von Wirk- und Hilfsstoffen): Kunststoffzahnstocher behandelt mit 38 IE Heparin/ml 10%iger Tween^{®}20 Lösung.
Primer: Primerpaar # 7 Soja, Primerpaar # 8 Mais, Primerpaar # 9 Weizen, Primerpaar # 10 Tabak
PCR-Programm: # 1 für Weizen und Tabak. # 4 für Soja und Mais
Das Ergebnis ist in Figur 11 gezeigt: M - Marker (1x je Gel); N - Negativkontrolle (No template control, 1x je Pflanzenart); A - Soja (4x); B - Mais (4x); C - Weizen (4x); D - Tabak (4x)
Fazit: Durch Verwendung von heparinbehandelter Zahnstocher zum Transfer von Blattmaterial on Soja, Mais, Weizen und Tabak lassen sich mit Pflanzenarten spezifischen Primern in der PCR Amplifikate erzeugen.

### Beispiel 12: Arabidopsis - Probennahme: durchstechen, kratzen

Probenmaterial: Arabidopsis Blätter
Werkzeugbeschreibung (Art und Menge von Wirk- und Hilfsstoffen): Kunststoffzahnstocher behandelt mit 38 IE Heparin/ml 10 %iger Tween^{®}20 Lösung
Primer: Primerpaar # 11
PCR-Programm: # 5
Das Ergebnis ist in Figur 12 gezeigt: M: Marker, DNA-Leiter; A: Alte Blätter mit heparinbehandeltem Zahnstocher zur Probenaufnahme durchstochen; B: Junge Blätter mit heparinbehandeltem Zahnstocher zur Probenaufnahme angekratzt; C: Junge Blätter mit heparinbehandeltem Zahnstocher zur Probenaufnahme durchstochen; N: Negativkontrolle (keine Probenzugabe)
Fazit: Von jungen und alten Arabidopsisblättern können mit Hilfe von heparinisierten Zahnstochern Proben in die PCR transferiert und dann erfolgreich amplifiziert werden. Die Pflanzenblattprobe kann sowohl mittels Durchstechung des Blattes oder mittels Ankratzen des Blattes aufgenommen werden.

### Beispiel 13: Tabak - Probennahme: anstechen, kratzen. Zeitdauer Probentransfer 0-4 s

Probenmaterial: Tabakblätter
Werkzeugbeschreibung (Art und Menge von Wirk- und Hilfsstoffen): Kunststoffzahnstocher behandelt mit 38 IE Heparin/ml 10 %iger Tween^{®}20 Lösung
Primer: Primerpaar # 10
PCR-Programm: # 1
Das Ergebnis ist in Figur 13 gezeigt: M: Marker, DNA-Leiter; A: Blätter mit heparinisiertem Zahnstocher angestochen, dann sofort Transfer in die PCR; B: Blätter mit heparinisiertem Zahnstocher angestochen, dann vier Sekunden warten, dann Transfer in die PCR; C: Blätter mit heparinisiertem Zahnstocher angekratzt, dann vier Sekunden warten, dann Transfer in die PCR; N: Negativkontrolle (keine Probenzugabe)
Fazit: Von Tabakblättern können mit Hilfe von heparinisierten Zahnstochern Proben in die PCR transferiert und dann erfolgreich amplifiziert werden. Die Pflanzenblattprobe kann nach Aufnahme auf den Zahnstocher durch Anstechen des Blattes sofort (ca. eine Sekunde) oder nach Wartezeit von 4 s in die PCR transferiert werden und liefert Amplifikate.

### Beispiel 14: Tabak - Probennahme und -transfer: stechen, kratzen, 1x, 2x antippen, einrühren

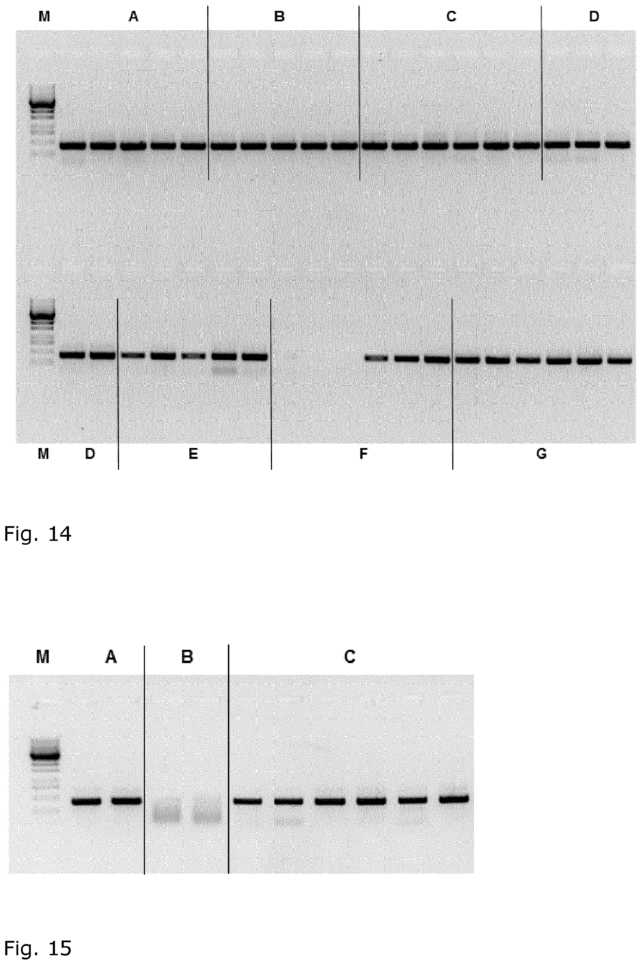

Probenmaterial: Tabakblatt
Werkzeugbeschreibung (Art und Menge von Wirk- und Hilfsstoffen): Kunststoffzahnstocher behandelt mit 38 IE Heparin/ml 10 %iger Tween^{®}20 Lösung
Primer: Primerpaar # 10
PCR-Programm: # 1
Das Ergebnis ist in Figur 14 gezeigt: M: Marker, DNA-Leiter; A: Blatt anstechen --> sofort Transfer (einmal Eindippen) in die PCR; B: Blatt anstechen --> nach 4 s Transfer in die PCR; C: Blatt anstechen --> sofort 2x Eindippen in die PCR; D: Blatt anstechen --> sofort in die PCR einrühren; E: Blatt ankratzen --> sofort in die PCR Eindippen; F: Blatt ankratzen --> sofort 2x in die PCR Eindippen; G: Blatt ankratzen --> sofort in die PCR einrühren
Fazit: Von Tabakblättern können mit Hilfe von heparinisierten Zahnstochern Proben in die PCR transferiert und dann erfolgreich amplifiziert werden. Nach Anstechen des Pflanzenblattes kann direkt oder erst nach 4 s der Zahnstocher in die PCR gedippt werden; dabei kann die Probe vom Zahnstocher durch einmaliges oder zweimaliges Eindippen der Spitze in die PCR oder durch Einrühren überführt werden. Ein Ankratzen der Blattoberfläche führt teilweise zu ausbleibender Amplifikation, wahrscheinlich, weil beim Kratzen im Vergleich zum Stechen weniger Pflanzenmaterial an der Spitze hängen bleibt.

### Beispiel 15: Mechanische Stabilität der Beschichtung. Tabak

Probenmaterial: Tabakblatt
Werkzeugbeschreibung (Art und Menge von Wirk- und Hilfsstoffen): Kunststoffzahnstocher behandelt mit 38 IE Heparin/ml 10%iger Tween^{®}20 Lösung
Primer: Primerpaar # 10
PCR-Programm: # 1
Das Ergebnis ist in Figur 15 gezeigt: M: Marker, DNA-Leiter; A: Heparin-beschichtete Zahnstocher zum Probentransfer verwendet, frisch, ohne schütteln; B: Unbeschichtete Zahnstocher zum Probentransfer verwendet.; C: Heparin-beschichtete Zahnstocher vor Verwendung in einer Verpackung (Kunststoffbeutel im Karton) geschüttelt (Transportsimulation)
Fazit: Heparinbehandelte Zahnstocher behalten ihre Funktionalität als inhibitorinaktivierende Probentransferwerkzeuge auch dann, wenn sie zuvor in einem Kunststoffbeutel einige Zeit geschüttelt werden (Transportsimulation).

### Beispiel 16: Duplex PCR Tabak-Wein, Bioanavlzer

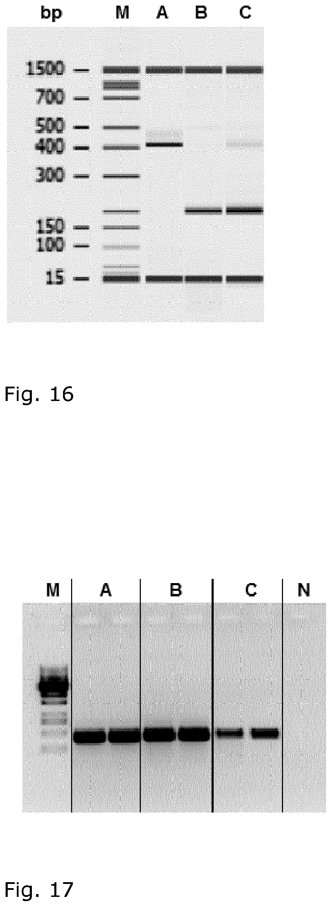

Probenmaterial: Blatt von Tabak, Wein
Werkzeugbeschreibung (Art und Menge von Wirk- und Hilfsstoffen): Kunststoffzahnstocher behandelt mit 38 IE Heparin/ml 10 %iger Tween^{®}20 Lösung
Primer: Primerpaar # 10; Primerpaar # 12
PCR-Programm: # 1
Das Ergebnis ist in Figur 16 gezeigt: M - Marker; A - Duplex PCR-Mix; Template: Tabak; B - Duplex PCR-Mix; Template: Weinblätter; C - Duplex PCR; Template: Tabak + Weinblätter
Fazit: Mit dem Werkzeug ist es auch möglich, Probenmaterial von zwei verschiedenen Pflanzen durch aufeinanderfolgenden Anstechen in eine PCR zu transferieren und erfolgreich zu amplifizieren.

### Beispiel 17: Soja, Baumwolle, Weizen, Amplikon 418 bp

Probenmaterial: Blatt von Soja, Baumwolle, Weizen
Werkzeugbeschreibung (Art und Menge von Wirk- und Hilfsstoffen): Kunststoffzahnstocher behandelt mit 38 IE Heparin/ml 10 %iger Tween^{®}20 Lösung
Primer: Primerpaar # 13
PCR-Programm: # 6
Das Ergebnis ist in Figur 17 gezeigt: M - Marker; A - Soja; B - Baumwolle; C - Weizen; N - Negativkontrolle (keine Probenzugabe)
Fazit: Von Soja-, Baumwolle- und Weizen-Blattmaterial kann bei Transfer von Probenmaterial mit dem Werkzeug erfolgreich ein 418 bp große Produkt amplifiziert werden.

### Beispiel 18: Baumwolle, Weizen, Amplikon 745 bp

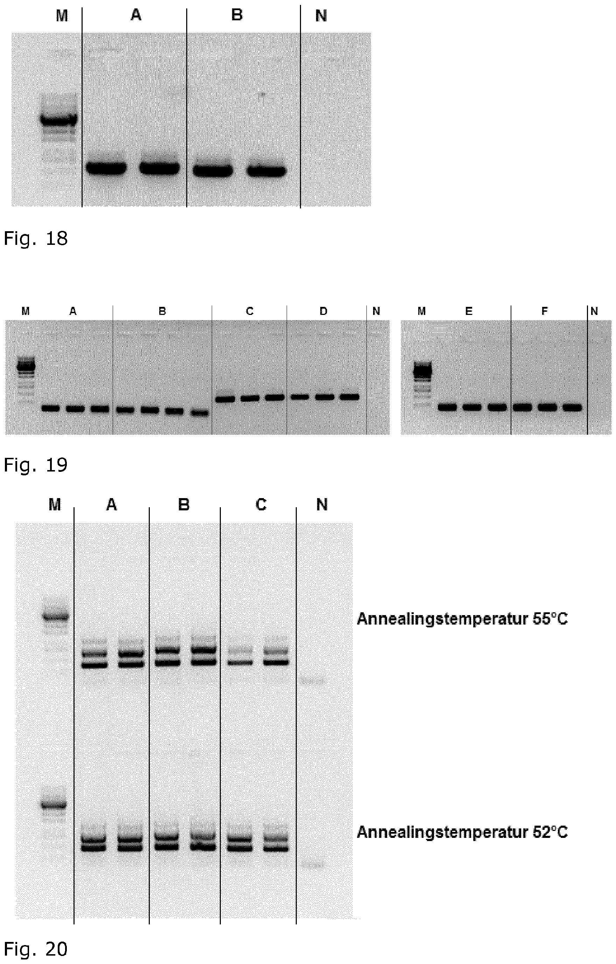

Probenmaterial: Blätter von Baumwolle und Weizen
Werkzeugbeschreibung (Art und Menge von Wirk- und Hilfsstoffen): Kunststoffzahnstocher behandelt mit 38 IE Heparin/ml 10 %iger Tween^{®}20 Lösung
Primer: Primerpaar # 14
PCR-Programm: # 7
Das Ergebnis ist in Figur 18 gezeigt: M: Maker, DNA-Leiter; A: Baumwolle; B: Weizen; N: Negativkontrolle (No template control)
Fazit: Nach Transfer von Baumwoll- und Weizenblattmaterial mittels des Werkzeugs in die PCR ist es möglich ein 745 bp großes DNA-Fragment zu amplifizieren.

### Beispiel 19: Tabak, Weinblätter: Nach Probenaufnahme direkter Proben-transfer vs. Eintrocknung Probe

Probenmaterial: Blattmaterial von Soja, Tabak, Wein
Werkzeugbeschreibung (Art und Menge von Wirk- und Hilfsstoffen): Kunststoffzahnstocher behandelt mit 38 IE Heparin/ml 10 %iger Tween^{®}20 Lösung
Primer: Primerpaar # 7 Soja, Primerpaaar # 10 Tabak, Primerpaar # 12 Wein
PCR-Programm: # 7 Tabak, Wein; # 8 Soja
Das Ergebnis ist in Figur 19 gezeigt: M: Marker, DNA-Leiter; A: Soja. Direkter Probentransfer nach Aufnahme der Probe auf das Werkzeug; B: Soja. Auf Werkzeug aufgetrocknete Probe: Nach Aufnahme der Probe auf das Werkzeug wurde es für 1,5 h an der Luft getrocknet. Erst danach erfolgte die Berührung der Werkzeugspitze mit der PCR; C: Tabak. Direkter Probentransfer nach Aufnahme der Probe auf das Werkzeug; D: Tabak. Auf Werkzeug aufgetrocknete Probe: Nach Aufnahme der Probe auf das Werkzeug wurde es für 1,5 h an der Luft getrocknet. Erst danach erfolgte die Berührung der Werkzeugspitze mit der PCR; E: Weinblätter. Direkter Probentransfer nach Aufnahme der Probe auf das Werkzeug; F: Weinblätter, Auf Werkzeug aufgetrocknete Probe: Nach Aufnahme der Probe auf das Werkzeug wurde es für 1,5 h an der Luft getrocknet. Erst danach erfolgte die Berührung der Werkzeugspitze mit der PCR; N - Negativkontrolle (keine Probenzugabe).
Fazit: Nach Aufnahme von Pflanzenblattmaterial auf das Werkzeug kann das Werkzeug für 1,5 h gelagert werden, bevor das Probenmaterial vom Werkzeug in die PCR überführt wird, ohne Beeinträchtigung der Funktionalität.

### Beispiel 20: Duplex PCR Soja, Baumwolle, Weizen, 418 bp + 757 bp

Probenmaterial: Blattmaterial von Soja, Baumwolle, Weizen
Werkzeugbeschreibung (Art und Menge von Wirk- und Hilfsstoffen): Kunststoffzahnstocher behandelt mit 38 IE Heparin/ml 10 %iger Tween^{®}20 Lösung
Primer: Primermix # 15
PCR-Programm: # 7 (Annealing 55 °C); # 11 (Annealing 52 °C)
Das Ergebnis ist in Figur 20 gezeigt: M: Marker, DNA-Leiter; A: Soja; B: Baumwolle; C: Weizen; N: Negativkontrolle (keine Probenzugabe)
Fazit: Mit Verwendung des Transferwerkzeuges ist es möglich, nachfolgend Duplex PCRs durchzuführen und in einer PCR sowohl ein 418 bp Fragment als auch ein 757 bp Fragment aus Pflanzenblattmaterial zu amplifizieren.

### Beispiel 21: Probennahme: Einstechen vs. Durchstechen, Tabak, Wein

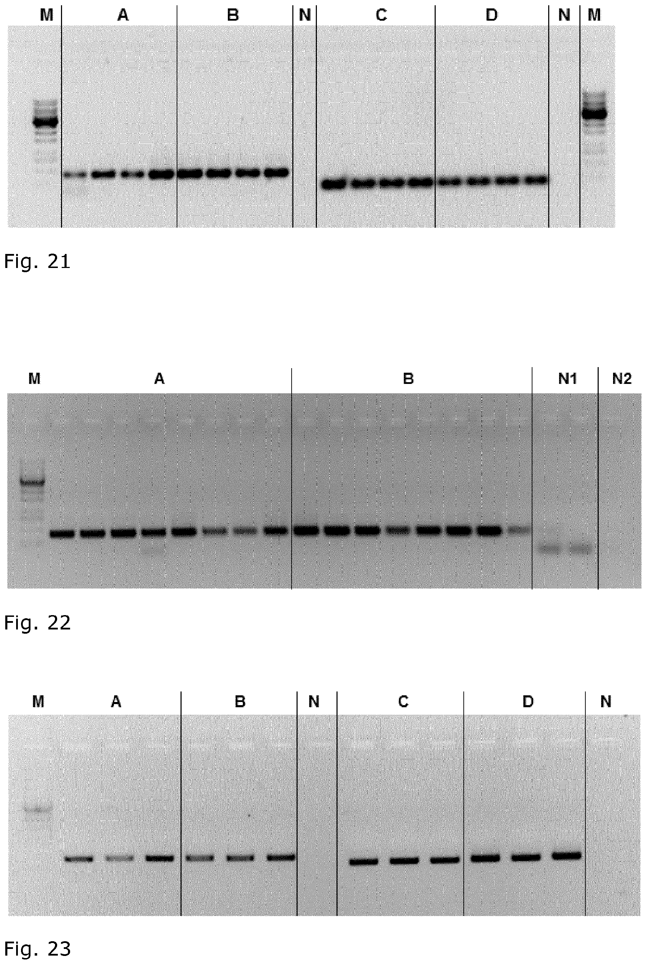

Probenmaterial: Blätter von Tabak und Wein
Werkzeugbeschreibung (Art und Menge von Wirk- und Hilfsstoffen): Kunststoffzahnstocher behandelt mit 38 IE Heparin/ml 10 %iger Tween^{®}20 Lösung
Primer: Primerpaar # 10 Tabak, Primerpaar # 12 Wein
PCR-Programm: # 9
Das Ergebnis ist in Figur 21 gezeigt: M: Marker, DNA-Leiter; A: Tabak. Einstechen - das Blatt liegt auf einer festen Unterlage und mit dem Transferwerkzeug wird in das Blatt hineingestochen; B: Tabak. Durchstechen - das Blatt wird mit dem Werkzeug ca. 5-10 mm durchstochen; C: Wein. Einstechen - das Blatt liegt auf einer festen Unterlage und mit dem Transferwerkzeug wird in das Blatt hineingestochen; D: Wein. Durchstechen - das Blatt wird mit dem Werkzeug ca. 5-10 mm durchstochen; N: Negativkontrolle (keine Probenzugabe)
Fazit: Mit dem Probentransferwerkzeug kann aus einen Pflanzenblatt Probenmaterial aufgenommen werden, indem in das Blatt hineingestochen wird, während es auf einer festen Unterlage liegt oder indem es 5 - 10 mm durchstochen wird.

### Beispiel 22: Beschichtungsprozedur: Einzeln oder im Bündel

Probenmaterial: Blatt vom Tabak, welches vor Verwendung ca. 1,5 Monate bei ca. 4 °C im Kühlschrank gelagert wurde
Werkzeugbeschreibung (Art und Menge von Wirk- und Hilfsstoffen): Kunststoffzahnstocher behandelt mit 38 IE Heparin/ml 10%iger Tween^{®}20 Lösung
Primer: Primerpaar # 10
PCR-Programm: # 1
Das Ergebnis ist in Figur 22 gezeigt: M: Marker, DNA-Leiter; A: Zahnstocher wurden einzeln beschichtet; B: Zahnstocher wurden als Bündel von 100 Stück beschichtet. Aus dem Bündel wurden von der Mitte und vom Rand zur Testung Zahnstocher zum Probentransfer verwendet; N1: Negativkontrolle 1 - Unbeschichtete Zahnstocher zum Probentransfer verwendet; N2: Negativkontrolle 2 (No template control)
Fazit: Zahnstocher könne sowohl einzeln, als auch gebündelt zu 100 Stück mit Heparin/Detergenz beschichtet werden, um zu der Funktionalität des Inhibitor inaktivierenden Probentransferwerkzeuges zu gelangen.

### Beispiel 23: Vergleich: Lysat vs. direkter Probentransfer. Tabak, Baumwolle

Probenmaterial: Blatt von Tabak und Baumwolle direkt, bzw. ein Lysat davon (Lysatherstellung: 20 µl Lysepuffer + 0,5 µl Proteinase K + 1 mm Durchmesser Blattstanzling, 3 min Inkubation bei Raumtemperatur, 3 min Inkubation bei 98 °C). Werkzeugbeschreibung (Art und Menge von Wirk- und Hilfsstoffen): Kunststoffzahnstocher behandelt mit 38 IE Heparin/ml 10 %iger Tween^{®}20 Lösung
Primer: Primerpaar # 10 Tabak (422 bp Amplifikat), Primerpaar # 5 Baumwolle (308 bp Amplifikat)
PCR-Programm: # 1
Das Ergebnis ist in Figur 23 gezeigt: M: Marker; A: Tabak, Probentransfer mit behandeltem Probentransferwerkzeug; B: Tabak; Lysatherstellung mit Lysepuffer und Proteinase, dann Transfer von 1 µl hiervon in die PCR; N: Negativkontolle (No template contol); C: Bauwolle; Probentransfer mit behandeltem Probentransferwerkzeug; D: Baumwolle; Lysatherstellung mit Lysepuffer und Proteinase, dann Transfer von 1 µl hiervon in die PCR; N: Negativkontolle (No template control)
Fazit: Bei Verwendung des mit Wirkstoff beschichteten Transferwerkzeuges kann die PCR genauso erfolgreich ablaufen wie bei Verwendung eines Pflanzenblattlysates als Probenmaterial.

### Beispiel 24: PCR-Volumen 10-50 ul, Tabak, Baumwolle

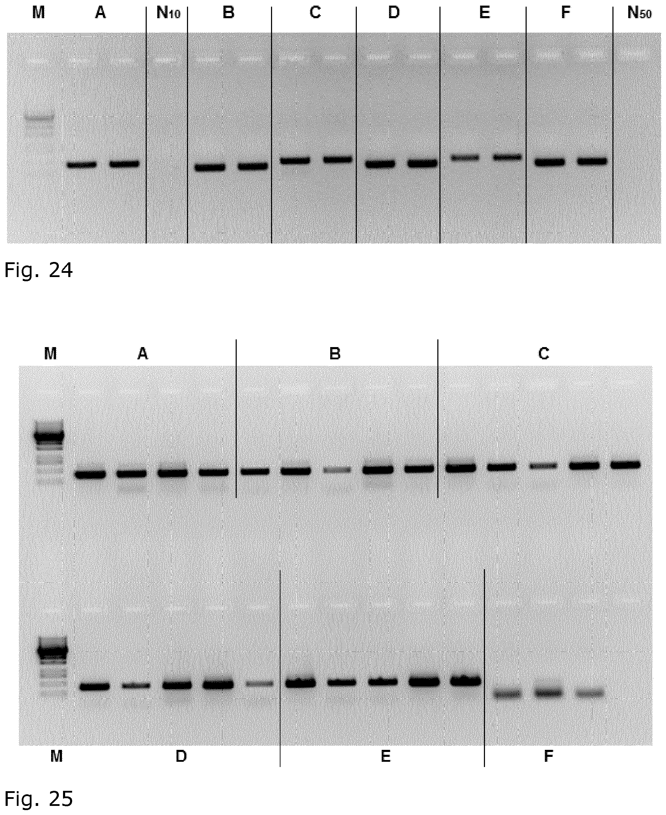

Probenmaterial: Blätter von Baumwolle, Tabak
Werkzeugbeschreibung (Art und Menge von Wirk- und Hilfsstoffen): Kunststoffzahnstocher behandelt mit 38 IE Heparin/ml 10 %iger Tween^{®}20 Lösung
Primer: Primerpaar # 10 Tabak, Primerpaar # 5 Baumwolle
PCR-Programm: # 1
Das Ergebnis ist in Figur 24 gezeigt: M: Marker, DNA-Leiter; A: PCR-Volumen 10 µl; Tabak; N10: PCR-Volumen 10 µl, Negativkontrolle (keine Probenzugabe); B: PCR-Volumen von 10 µl; Baumwolle; C: PCR-Volumen 20 µl, Tabak; D: PCR-Volumen 20 µl, Baumwolle; E: PCR-Volumen 50 µl; Tabak; F: PCR-Volumen 50 µl; Baumwolle; N50: PCR-Volumen 50 µl, Negativkontrolle (keine Probenzugabe)
Fazit: Mit Wirkstoff beschichtete Probentransferwerkzeuge können verwendet werden um Probenmaterial in 10 µl-50 µl Volumen umfassende PCRs zu transferieren und ermöglichen so die erfolgreiche PCR-Amplifikation der Zielprodukte.

### Beispiel 25: Lagerversuch der Beschichtung: frisch vs. 2 Monate bei -20°C bis +37°C

Probenmaterial: Blatt vom Tabak
Werkzeugbeschreibung (Art und Menge von Wirk- und Hilfsstoffen): Kunststoffzahnstocher behandelt mit 38 IE Heparin/ml 10 %iger Tween^{®}20 Lösung
Primer: Primerpaar # 10
PCR-Programm: # 12
Das Ergebnis ist in Figur 25 gezeigt: M: Marker, DNA-Leiter; A: Verwendung von frisch hergestellten Transferwerkzeugen; B: Verwendung von beschichteten Transferwerkzeugen, die 2 Monate bei RT gelagert wurden; C: Verwendung von beschichteten Transferwerkzeugen, die 2 Monate bei 37 °C gelagert wurden; D: Verwendung von beschichteten Transferwerkzeugen, die 2 Wochen bei 4 °C gelagert wurden; E: Verwendung von beschichteten Transferwerkzeugen, die 2 Wochen bei -20 °C gelagert wurden; F: Verwendung von unbeschichteten Zahnstochern
Fazit: Die mit Heparin/Detergenz beschichteten Transferwerkzeuge sind für mindestens 2 Monate bei 37 °C-Lagerung stabil und können danach als Inhibitor inaktivierende Probentransferwerkzeuge verwendet werden.

### Beispiel 26: Produktvergleich

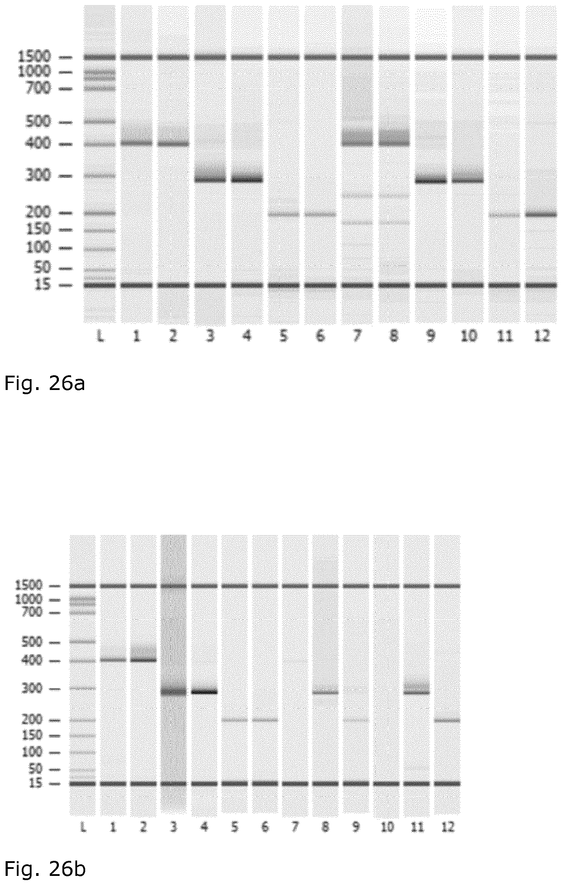

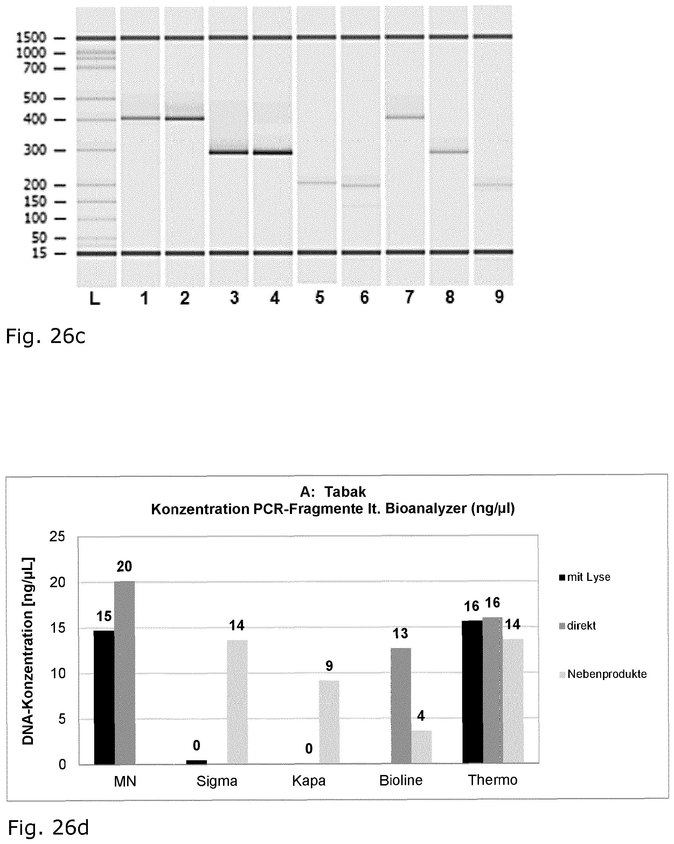

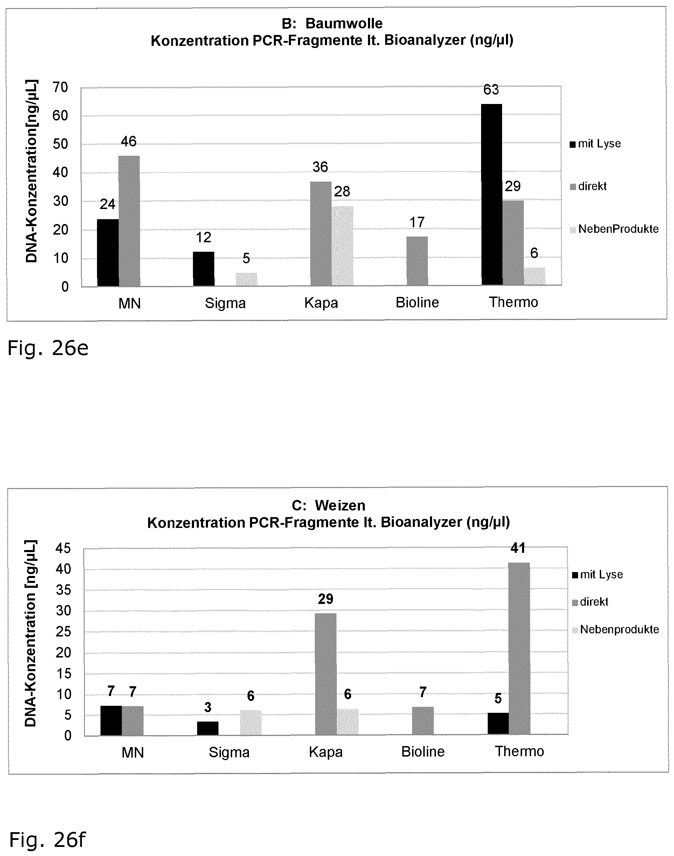

Probenmaterial: Blätter von Weizen, Tabak, Baumwolle
Werkzeugbeschreibung (Art und Menge von Wirk- und Hilfsstoffen): Kunststoffzahnstocher behandelt mit 38 IE Heparin/ml 10%iger Tween^{®}20 Lösung
Primer: Primerpaar # 9 Weizen, Primerpaar # 10 Tabak, Primerpaar # 5 Baumwolle
PCR-Programm in Eppendorf Gradient Cycler: MN: PCR Programm # 1; Thermo Scientific: # 13; Sigma: # 14; Bioline: # 15; KAPA: # 16
Das Ergebnis ist in Figur 26 a - f gezeigt. Es wurden folgende Prozeduren/Produkte miteinander verglichen: MN, einfache Probenvorbereitung mittels schneller Lyse; MN, erfindungsgemäßes Verfahren mittels Probentransferwerkzeug; Thermo Scientific Phire Plant Direct PCR, Verdünnungsprotokoll; Thermo Scientific Phire Plant Direct PCR, Direktprotokoll, Probentransfer mittels Biopsypuncher; Sigma Extrac-N-Amp Plant PCR, einfache Probenvorbereitung mittels schneller Lyse; Bioline MyTaq Plant PCR, Direktprotokoll; KAPA3G Plant PCR, Direktprotokoll
Alle Prozeduren / Kits wurden mit Blättern von Weizen, Tabak und Baumwolle getestet.

Die Durchführung der Probenvorbereitung mittels schneller Lyse erfolgte wie folgt: 20 µl Lysepuffer enthaltend Tris, KCl sowie nichtionisches Detergenz) + 0,5 µl Proteinase K Lösung (20 mg/ml) + ca. 1qmm Blattscheibe, 2 min bei Raumtemperatur schütteln, 3 min bei 98 °C inkubieren. Hiervon wurde dann 1 µl als Template in die PCR gegeben.

Die Durchführung mit den Produkten von Thermo, Sigma, Bioline und Kapa erfolgten laut Herstellerangaben. Die Amplifikate wurden mit Bioanalyzer untersucht.

Vergleich gegen Thermo Scientific Phire Plant Direct PCR (Fig. 26 a)
- L:: DNA-Leiter
- 1:: MN, Tabak, Probenvorbereitung mittels schneller Lyse
- 2:: MN, Tabak, Verwendung des erfindungsgemäßen Probentransferwerkzeuges
- 3:: MN, Baumwolle, Probenvorbereitung mittels schneller Lyse
- 4:: MN, Baumwolle, Verwendung des erfindungsgemäßen Probentransferwerkzeuges
- 5:: MN, Weizen, Probenvorbereitung mittels schneller Lyse
- 6:: MN, Weizen, Verwendung des erfindungsgemäßen Probentransferwerkzeuges
- 7:: Thermo, Tabak, Verdünnungsprotokoll
- 8:: Thermo, Tabak, Probentransfer mittels Biopsypuncher
- 9:: Thermo, Baumwolle, Verdünnungsprotokoll
- 10:: Thermo, Baumwolle, Probentransfer mittels Biopsypuncher
- 11:: Thermo, Weizen, Verdünnungsprotokoll
- 12:: Thermo, Weizen, Probentransfer mittels Biopsypuncher

Fazit: In allen Fällen werden die Zielfragmente (422 bp, 308 bp, 201 bp) amplifiziert. Das erfindungsgemäße Verfahren (Spur 2, 4, 6 Verwendung des Probentransferwerkzeugs) ist gegenüber der Methode Thermo Scientific Phire Plant Direct PCR, Direktprotokoll, Probentransfer mittels Biopsypuncher, insofern vorteilhaft, als das kein Biopsie-Stanzer verwendet und gereinigt / dekontaminiert werden muss und sich darüber hinaus weniger Nebenprodukte bilden.

Vergleich gegen Sigma Extrac-N-Amp Plant PCR und KAPA3G Plant PCR, Direktprotokoll (Fig. 26 b)
- L:: DNA-Leiter
- 1:: MN, Tabak, Probenvorbereitung mittels schneller Lyse
- 2:: MN, Tabak, Verwendung des erfindungsgemäßen Probentransferwerkzeuges
- 3:: MN, Baumwolle, Probenvorbereitung mittels schneller Lyse
- 4:: MN, Baumwolle, Verwendung des erfindungsgemäßen Probentransferwerkzeuges
- 5:: MN, Weizen, Probenvorbereitung mittels schneller Lyse
- 6:: MN, Weizen, Verwendung des erfindungsgemäßen Probentransferwerkzeuges
- 7:: Sigma, Tabak, einfache Probenvorbereitung mittels schneller Lyse
- 8:: Sigma, Baumwolle, einfache Probenvorbereitung mittels schneller Lyse
- 9:: Sigma, Weizen, einfache Probenvorbereitung mittels schneller Lyse
- 10:: Kapa, Tabak, Probentransfer mittels Biopsypuncher
- 11:: Kapa, Baumwolle, Probentransfer mittels Biopsypuncher
- 12:: Kapa, Weizen, Probentransfer mittels Biopsypuncher

Fazit: In den meisten Fällen werden die Zielfragmente (422 bp, 308 bp, 201 bp) amplifiziert. Das erfindungsgemäße Verfahren (Spur 2, 4, 6 Verwendung des Probentransferwerkzeugs) ist gegenüber der Methode Sigma und Kapa insofern vorteilhaft, als das auch das 422 bp Produkt gebildet wird, dieses jedoch mit dem Sigma und Kapa Kit nicht gebildet wird (Spur 7, 10). Weiterhin ist das erfindungsgemäße Verfahren gegen über den andere beiden Kits vorteilhaft, dass entweder kein Biopsie-Stanzer verwendet und gereinigt/dekontaminiert werden muss oder kein Lysat hergestellt werden muss.

Vergleich gegen Bioline MyTaq Plant PCR, Direktprotokoll (Fig. 26 c)
- L:: Marker
- 1:: MN, Tabak, Probenvorbereitung mittels schneller Lyse
- 2:: MN, Tabak, Verwendung des erfindungsgemäßen Probentransferwerkzeuges
- 3:: MN, Baumwolle, Probenvorbereitung mittels schneller Lyse
- 4:: MN, Baumwolle, Verwendung des erfindungsgemäßen Probentransferwerkzeuges
- 5:: MN, Weizen, Probenvorbereitung mittels schneller Lyse
- 6:: MN, Weizen, Verwendung des erfindungsgemäßen Probentransferwerkzeuges
- 7:: Bioline, Tabak,
- 8:: Bioline, Baumwolle,
- 9:: Bioline, Weizen,

Fazit: In allen Fällen werden die Zielfragmente (422 bp, 308 bp, 201 bp) amplifiziert. Das erfindungsgemäße Verfahren (Spur 2, 4, 6, Verwendung des Probentransferwerkzeugs) ist gegenüber der Methode Bioline insofern vorteilhaft, als das kein Biopsie-Stanzer verwendet und gereinigt / dekontaminiert werden muss und mehr Zielprodukt gebildet wurde.

Die quantitative Auswertung der gebildeten Amplifikate ist in nachfolgenden Abbildungen dargestellt.

Abbildung: Fig. 26 d Tabak; Fig. 26 e Baumwolle; Fig. 26 f Weizen:
Beschriftung: MN, schwarzer Balken: Lyseprotokoll; MN, grauer Balken: Verwendung des den Inhibitor inaktivierende Probentransfer-Werkzeugs; Sigma: Sigma Extrac-N-Amp Plant PCR Kit; Kapa: KAPA3G Plant PCR Kit; Bioline: Bioline MyTaq Plant PCR Kit; Thermo: Thermo Scientific Phire Plant Direct PCR Kit
Balken: schwarz: Lyseprotokoll; grau: Direkt = direkter Transfer von Blattmaterial in die PCR; hellgrau: Nebenprodukte
Fazit: Das erfindungsgemäße Verfahren (MN, direkt) liefert in der Mehrzahl der Fälle höhere Ausbeuten an Zielfragment, als die Mitbewerberverfahren. Weiterhin treten beim erfindungsgemäßen Verfahren weniger detektierbare Nebenprodukte auf (Amplifikate anderer Größen als die Zielgrößen).

### Versuchsgruppe III: Andere Probenarten

### Beispiel 27: Avocadofruchtfleisch: Werkzeug +/- Heparin

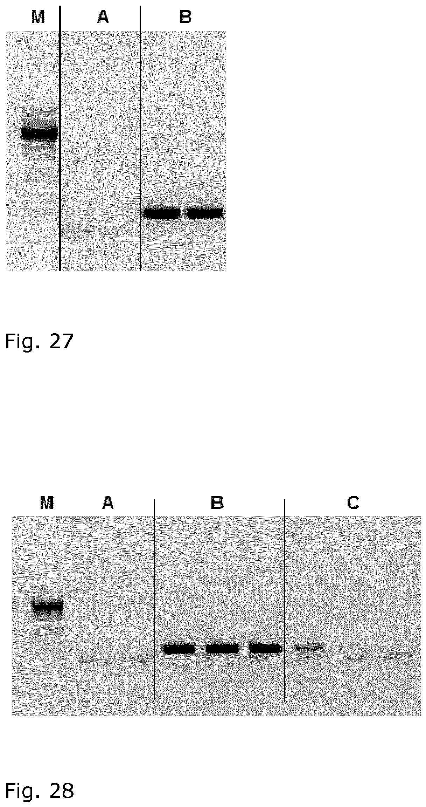

Probenmaterial: Fruchtfleisch von Avocado
Werkzeugbeschreibung (Art und Menge von Wirk- und Hilfsstoffen): Kunststoffzahnstocher behandelt mit 38 IE Heparin/ml 10%iger Tween^{®}20 Lösung
Primer: Primerpaar # 5
PCR-Programm: # 1
Das Ergebnis ist in Figur 27 gezeigt: M: Marker, DNA-Leiter; A: Unbehandelte Zahnstocher zum Probentransfer verwendet; B: Inhibitor inaktivierendes Probentransferwerkzeug verwendet
Fazit: Bei Verwendung des mit Wirkstoff beschichteten Zahnstochers (Probentransferwerkzeug) zum Transfer von Avocadofruchtfleisch als Probenmaterial in die PCR bildet sich gut erkennbares Zielprodukt, wohingegen bei Verwendung von unbehandelten Zahnstochern kein nennenswertes Amplifikat gebildet wird. Das erfindungsgemäße Verfahren ist somit nicht nur für Blut- und Pflanzenblattmaterial vorteilhaft verwendbar, sondern auch für Avocadofruchtfleisch.

### Beispiel 28: Avocadofruchtfleisch: Werkzeug +/- Heparin

Probenmaterial: Fruchtfleisch von Avocado
Werkzeugbeschreibung (Art und Menge von Wirk- und Hilfsstoffen): Kunststoffzahnstocher behandelt mit 38 IE Heparin/ml 10 %iger Tween^{®}20 Lösung
Primer: Primerpaar # 5
PCR-Programm: # 1
Das Ergebnis ist in Figur 28 gezeigt: M: Marker, DNA Leiter; A: Verwendung unbehandelter Zahnstocher zum Probentransfer; B: Verwendung behandelter Zahnstocher zum Probentransfer. Das Avocadofruchtfleisch wurde kurz angestochen; C: Verwendung behandelter Zahnstocher zum Probentransfer. Das Avocadofruchtfleisch wurde kurz angestochen sowie darüber gekratzt.
Fazit: Fazit: Bei Verwendung des mit Wirkstoff beschichteten Zahnstochers (Probentransferwerkzeug) zum Transfer von Avocadofruchtfleisch als Probenmaterial in die PCR bildet sich gut erkennbares Zielprodukt, wohingegen bei Verwendung von unbehandelten Zahnstochern kein nennenswertes Amplifikat gebildet wird. Wird das Probenmaterial nicht nur angestochen, sondern auch noch darüber mit dem Probentransferwerkzeug gekratzt, wird weniger oder kein Zielprodukt gebildet, was auf die größere Menge des transferierten Probenmaterials zurückzuführen ist.

### Beispielgruppe IV: Unterschiedliche Wirkstoffe

### Beispiel 29: Chondrotinsulfat 5%, 1% im Vergleich zu Heparin und Negativkontrolle

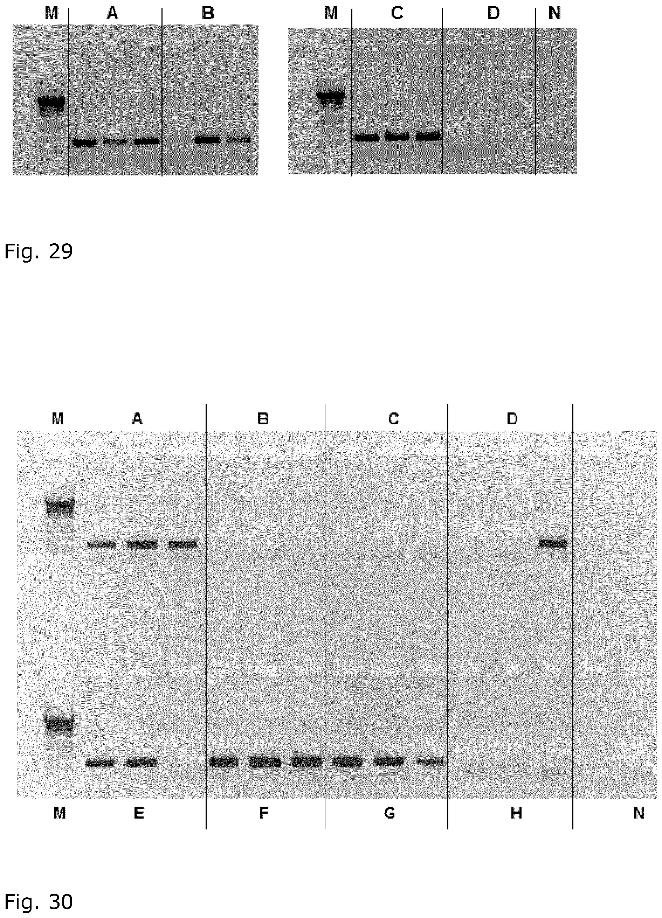

Probenmaterial: Maus EDTA-Blut
Werkzeugbeschreibung (Art und Menge von Wirk- und Hilfsstoffen): Kunststoffzahnstocher beschichtet mit: A: 5 %iger und B: 1 %iger Lösung von Chondrotinsulfat-Natriumsalz (CAS 9082-07-9) in einer 10 %igen Tween^{®}20-Lösung; C: 20 IE Heparin/ml 10 %iger Tween^{®}20 Lösung
Primer: Primerpaar # 2
PCR-Programm: # 2
Das Ergebnis ist in Figur 29 gezeigt: M: Marker, DNA-Leiter; Transfer der Probe mit Zahnstocher beschichtet mit: A: 5 %ige Lösung von Chondrotinsulfat-Natriumsalz in einer 10 %igen Tween^{®}20-Lösung, B: 1 %ige Lösung von Chondrotinsulfat-Natriumsalz in einer 10 %igen Tween^{®}20-Lösung, C: 20 IE Heparin je ml 10 %iger Tween^{®}20 Lösung, D: unbeschichteter Zahnstocher, N: Negativkontrolle (No template control)
Fazit: Nicht nur Heparin, sondern auch Chondroitinsulfat ist geeignet, um ein funktionelles Inhibitor inaktivierendes Probentransferwerkzeug herzustellen.

### Beispiel 30: Dextransulfat 0,1-5% im Vergleich zur Heparinkontrolle und NK, Maus EDTA-Blut

Probenmaterial: Maus EDTA-Blut
Werkzeugbeschreibung (Art und Menge von Wirk- und Hilfsstoffen): Kunststoffzahnstocher beschichtet mit einer Lösung von 5% Chodroitinsulfat bzw. 5%, 3%, 1%, 0,5%, 0,1% Dextransulfat (CAS 9011-18-1) in 10 %iger Tween^{®}20 Lösung. Kunststoffzahnstocher behandelt mit 38 IE Heparin/ml 10 %iger Tween^{®}20 Lösung.
Primer: Primerpaar # 2
PCR-Programm: # 2
Das Ergebnis ist in Figur 30 gezeigt: M: Marker, DNA-Leiter; Transfer der Probe mit Zahnstocher beschichtet mit: A: 5 %iger Lösung Chondrotinsulfat-Natriumsalz in 10 %iger Tween^{®}20 Lösung, B: 5 %iger Lösung Dextransulfat in 10%iger Tween^{®}20 Lösung, C: 3 %iger Lösung Dextransulfat in 10 %iger Tween^{®}20 Lösung, D: 1 %iger Lösung Dextransulfat in 10 %iger Tween^{®}20 Lösung, E: 0,5 %iger Lösung Dextransulfat in 10 %iger Tween^{®}20 Lösung, F: 0,1 %iger Lösung Dextransulfat in 10 %iger Tween^{®}20 Lösung, G: 20 IE Heparin / ml 10%iger Tween^{®}20 Lösung, H: Negativkontrolle unbeschichteter Zahnstocher, N: Negativkontrolle (No template control)
Fazit: Zahnstocher, die mit einer 0,1-0,5 %igen Lösung von Dextansulfat beschichtet sind, können als funktionelle Inhibitor inaktivierende Probentransferwerkzeuge verwendet werden (E, F), ebenso wie Zahnstocher, die mit Chondroitinsulfat-Natriumsalz (A) oder Heparin (G) beschichtet sind. Probentransfer mit unbehandelten Zahnstochern führt hingegen nicht zu einer erfolgreichen Amplifikation (H).

### Beispiel 31: Chaotropsalzlösung anstelle von Heparin

Probenmaterial: Kresseblattbrei, hergestellt durch Zerreibung von 1 Masseteil Kresseblätter mit 1 Masseteil Wasser
Werkzeugbeschreibung (Art und Menge von Wirk- und Hilfsstoffen): Kunststoffzahnstocher behandelt mit einer Lösung aus 6 M Guanidinhydrochlorid (CAS 50-01-01), 90 mM EDTA (CAS 60-00-4), 1,5% Tween^{®}20, 50 mM Tris-Cl pH 8) sowie Kunststoffzahnstocher behandelt mit 38 IE Heparin/ml 10 %iger Tween^{®}20 Lösung
Primer: Primerpaar # 5 für eine 308 bp Target; Primerpaar # 5 für ein 447 bp Target.
PCR-Programm: # 2
Das Ergebnis ist in Figur 30 gezeigt:
   Abbildung oberer Teil: Primerpaar # 5, 308 bp Amplifikat: M: Marker; A: Unbehandelte Zahnstocher zum Probentransfer; B: Mit Heparin behandelte Zahnstocher zum Probentransfer; C: Mit Chaotropsalzlösung behandelter Zahnstocher zum Probentransfer; N: Negativkontrolle (No template control)
   Abbildung unterer Teil: Primerpaar # 5, 447 bp Amplifikat: M: Marker; D: Unbehandelte Zahnstocher zum Probentransfer; E: Mit Heparin behandelte Zahnstocher zum Probentransfer; F: Mit Chaotropsalzlösung behandelter Zahnstocher zum Probentransfer; N: Negativkontrolle (No template control)
Fazit: Eine Inhibitor-inaktivierende Wirkung lässt sich für Kresseblattbrei nicht nur durch eine Beschichtung eines Probentransferwerkzeuges mit Heparin, sondern auch durch Beschichtung mit einer Chaotropsalzlösung erzeugen.

Anmerkung: Der Wirkmechanismus kann für Heparin und Chaotropsalzlösung gleich oder unterschiedlich sein. Es ist davon auszugehen, dass Heparin Inhibitoren in Proben bindet und dadurch inaktiviert. Chaotropsalze inhibieren entweder durch ihre denaturierende Wirkung Inhibitoren, sie können jedoch auch durch ihre denaturierende und lysefördernde Wirkung die DNA-Freisetzung unterstützen.

### Beispiel 32: Carrageenan 0,05 - 0,5%, Kaninchenblut

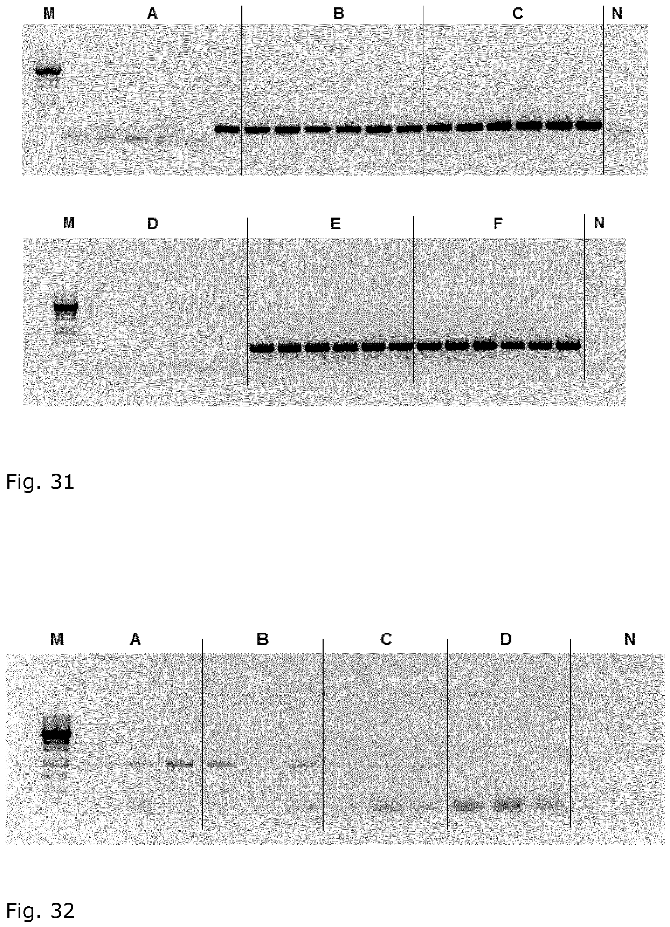

Probenmaterial: EDTA-Kaninchenblut
Werkzeugbeschreibung (Art und Menge von Wirk- und Hilfsstoffen): Kunststoffzahnstocher beschichtet mit 0,5 % bzw. 0,1 % bzw. 0,05 % λ-Carrageenan (CAS 9064-57-7) in Wasser
Primer: Primerpaar # 1
PCR-Programm: # 2
Das Ergebnis ist in Figur 32 gezeigt: M: Marker, DNA-Leiter; A: Probentransferwerkzeug beschichtet mit 0,5 % wässriger Lösung von λ-Carrageenan; B: Probentransferwerkzeug beschichtet mit 0,1 % wässriger Lösung von λ-Carrageenan; C: Probentransferwerkzeug beschichtet mit 0,05 % wässriger Lösung von λ-Carrageenan; D: Unbehandeltes Probentransferwerkzeug; N: Negativkontrolle (No template control)
Fazit: Transfer einer EDTA-Kaninchenblutprobe in eine 10 µl PCR mittels eines Kunststoffzahnstochers, der mit 0,5 %iger wässriger λ-Carrageenan Lösung beschichtet wurde, ermöglicht eine nachfolgende Amplifikation eines 813 bp langen DNA-Fragmentes, wohingegen die Verwendung eines unbehandelten Zahnstochers nicht zur Amplifikation des Zielfragmentes führt.

### Beispiel 33: Dextransulfat 0,01 %-1,5 % mit Tabak

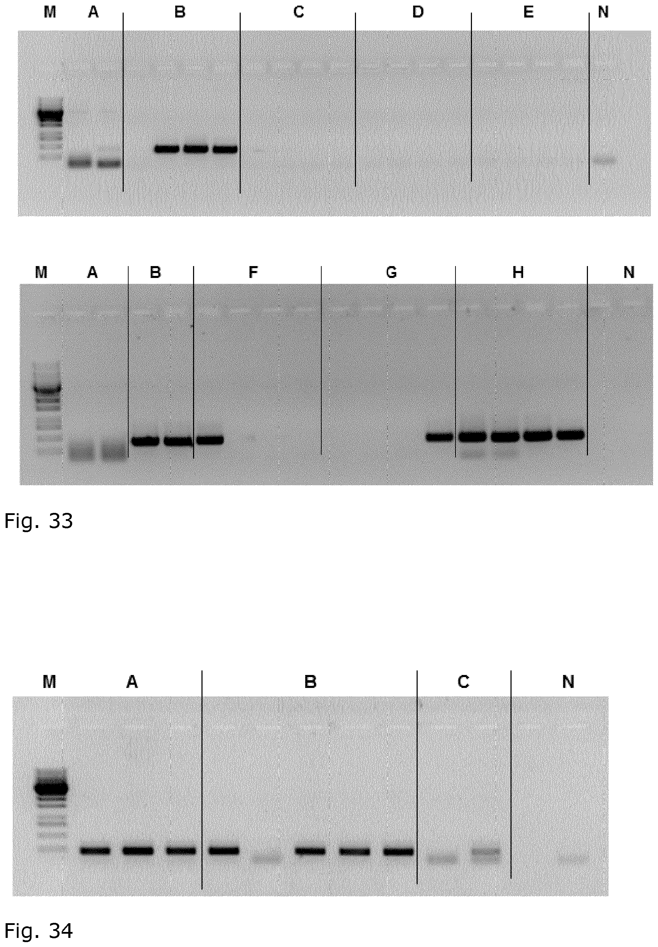

Probenmaterial: Tabakblatt, zwei Wochen im Kühlschrank gelagert
Werkzeugbeschreibung (Art und Menge von Wirk- und Hilfsstoffen): Kunststoffzahnstocher behandelt mit 38 IE Heparin/ml 10 %iger Tween^{®}20 Lösung bzw. mit Dextransulfatkonzentrationen von 0,01 %-1,5 % in 10 %iger Tween^{®}20 Lösung.
Primer: Primerpaar # 10
PCR-Programm: # 1
Das Ergebnis ist in Figur 33 gezeigt: M: Marker, DNA-Leiter; A: Unbehandelte Zahnstocher; B: Heparin-behandelte Zahnstocher; C: 0,5 % Dextransulfatlösung zur Zahnstocherbehandlung; D: 1% Dextransulfatlösung zur Zahnstocherbehandlung; E: 1,5 % Dextransulfatlösung zur Zahnstocherbehandlung; F: 0,1 % Dextransulfatlösung zur Zahnstocherbehandlung; G: 0,05 % Dextransulfatlösung zur Zahnstocherbehandlung; H: 0,01 % Dextransulfatlösung zur Zahnstocherbehandlung; N: Negativkontrolle (No template control)
Fazit: Durch Beschichtung von Zahnstochern mit einer 0,01 %igen Dextransulfatlösung lässt sich ein funktionelles Inhibitor-inaktivierendes Probentransferwerkzeug herstellen, welches erfolgreich für den Probentransfer/Amplifikation von Tabakblättern verwendet werden kann.

### Beispiel 34: Arabidopsis: Heparin, Dextran vs. Unbeschichtet und NK

Probenmaterial: Blätter von Arabidopsis
Werkzeugbeschreibung (Art und Menge von Wirk- und Hilfsstoffen): Kunststoffzahnstocher behandelt mit 38 IE Heparin/ml 10 %iger Tween^{®}20 Lösung bzw. mit Dextransulfatkonzentrationen von 0,01 %iger Tween^{®}20 Lösung.
Primer: Primerpaar # 11
PCR-Programm: # 1 jedoch mit 52 °C Annealing Temperatur
Das Ergebnis ist in Figur 34 gezeigt: M: Marker, DNA-Leiter; A: Heparin-beschichtete Zahnstocher; B: Dextransulfat (0,01 %) beschichtete Zahnstocher; C: Unbeschichtete Zahnstocher; N: Negativkontrolle (No templater control)
Fazit: Dextransulfat beschichtete Probentransferwerkzeuge können nicht nur für Tabakblätter sondern auch für Arabidopsisblätter erfolgreich zum Probentransfer verwendet werden.

### Beispiel 35: Mais: Dextran 0,001 %, 0,005 %, 0,01 % vs. unbeschichtet

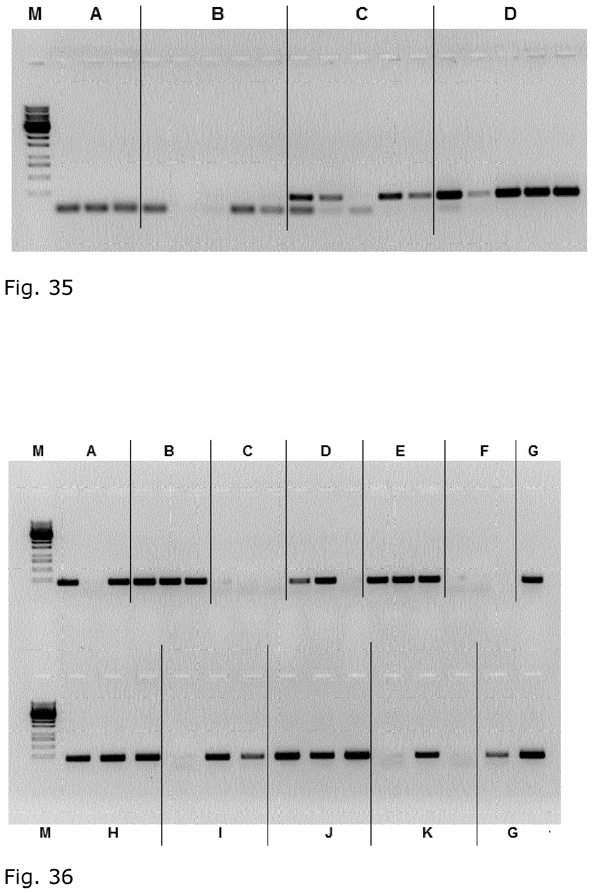

Probenmaterial: Blattmaterial von Mais
Werkzeugbeschreibung (Art und Menge von Wirk- und Hilfsstoffen): Kunststoffzahnstocher behandelt mit Dextransulfat-Natriumsalz in 10% Tween^{®}20-Lösung
Primer: Primerpaar # 8
PCR-Programm: # 4
Das Ergebnis ist in Figur 35 gezeigt: M: Marker - DNA-Leiter; A: Unbeschichteter Zahnstocher zum Probentransfer; B: Zahnstocher beschichtet mit 0,001 % Dextransulfat-Natriumsalz in 10 % Tween^{®}20; C: Zahnstocher beschichtet mit 0,005 % Dextransulfat-Natriumsalz in 10 % Tween^{®}20; D: Zahnstocher beschichtet mit 0,01 % Dextransulfat-Natriumsalz in 10 % Tween^{®}20
Fazit: Bei Verwendung von unbeschichteten Zahnstochern zum Transfer von Mais-Blattmaterial in die PCR, werden nur unspezifische Nebenprodukte amplifiziert. Werden Zahnstocher verwendet, die mit 0,005 % oder 0,01 % Dextransulfat beschichtet sind, wird in der PCR das Zielprodukt von 226 bp gebildet.

### Beispiel 36: GITC und GuHCl +/- Tween mit Weinblatt

Probenmaterial: Weinblätter
Werkzeugbeschreibung (Art und Menge von Wirk- und Hilfsstoffen): Kunststoffzahnstocher behandelt mit verschiedenen Chaotropsalzen
Primer: Primerpaar # 12
PCR-Programm: # 12
Das Ergebnis ist in Figur 36 gezeigt: M: Marker, DNA-Leiter; A: Zahnstocherbeschichtung: 4 M GuSCN in 2 % Tween^{®}20; B: Zahnstocherbeschichtung: 4 M GuSCN in 10 % Tween^{®}20; C: Zahnstocherbeschichtung: 5 M GuSCN; D: Zahnstocher-beschichtung: 5 M GuSCN in 10 % Tween^{®}20; E: Zahnstocherbeschichtung: LiHeparin-Tween^{®}; F: Negativkontrolle: Zahnstocher unbeschichtet; G: Zahnstocher-beschichtung: 8 M GuHCl in 10 % Tween^{®}20; H: Zahnstocherbeschichtung: 6,4 M GuHCl in 20 % Tween^{®}20; I: Zahnstocherbeschichtung: 6,5 M GuHCl; J: Zahnstocher-beschichtung: 6,5 M GuHCl in 10 % Tween^{®}20; K: Zahnstocherbeschichtung: 8 M GuHCl
Fazit: Werden Zahnstocher zum Probentransfer verwendet, die mit Chaotropsalz/Tween^{®}20 Mischungen beschichtet wurden, kann dies zu vergleichbar guter Amplifikation von DNA in der PCR führen, wie bei Verwendung von Heparin/Tween^{®}20 beschichteten Zahnstochern. Bei Verwendung unbeschichteter Zahnstocher zum Probentransfer von Weinblattmaterial wird hingegen kein Amplifikat gebildet (F).

### Beispiel 37: Tween^{®} als Hilfsstoff ersetzt durch Wasser bzw. Propylenglykol mit Blätter und Blut

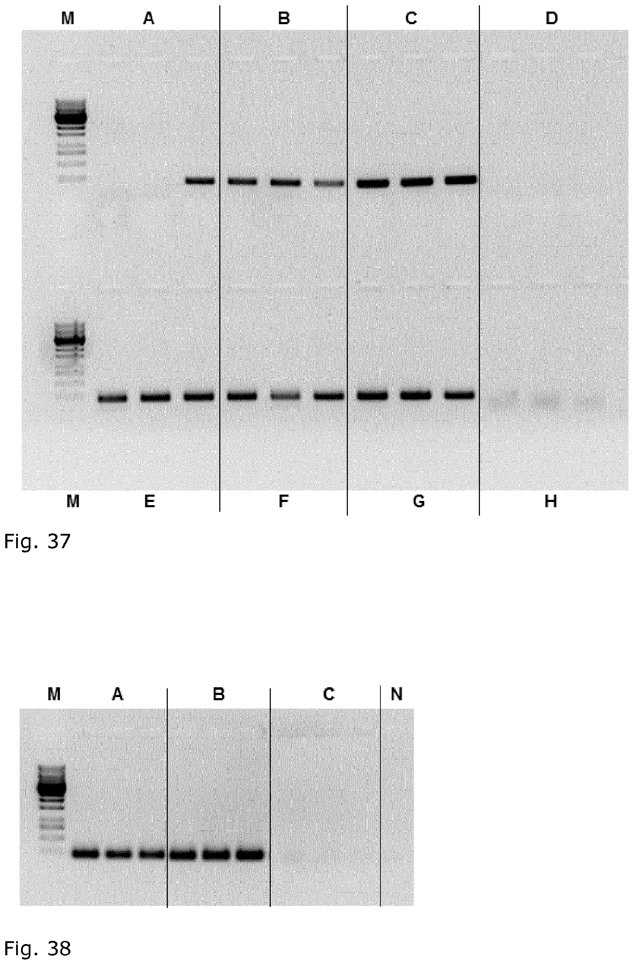

Probenmaterial: Weinblätter, Rattenblut
Werkzeugbeschreibung (Art und Menge von Wirk- und Hilfsstoffen): Kunststoffzahnstocher behandelt mit Heparin-Tween^{®}20, Heparin, Heparin-Polypropylenglykol. (Polypropylenglykol: CAS 25322-69-4, durchschnittliches Molekulargewicht: 2700 g/mol)
Primer: Primerpaar # 12 (Weinblätter), Primerpaar # 3 (Rattenblut)
PCR-Programm: # 12 (Weinblättr); # 17 (Rattenblut)
Das Ergebnis ist in Figur 37 gezeigt: M: Marker - DNA-Leiter; Zum Probentransfer für unterschiedlichen Probenmaterialien wurden Zahnstocher verwendet, die zuvor mit verschiedenen Lösungen beschichtet wurden; A: Weinblatt; Beschichtung: Heparin in Wasser; B: Weinblatt; Beschichtung: Heparin in 10 % Polypropylenglykol; C: Weinblatt; Beschichtung: Heparin in 10 % Tween^{®}20; D: Weinblatt; Beschichtung; keine; E: Blut; Beschichtung: Heparin in Wasser; F: Blut; Beschichtung: Heparin in 10 % Polypropylenglykol; G: Blut; Beschichtung: Heparin in 10 % Tween^{®}20; H: Blut; Beschichtung; keine
Fazit: Die Verwendung eines viskosen, nicht trocknenden Hilfsstoffes zu Lösung und zum Auftrag des Wirkstoffs Heparin auf einen Zahnstocher verbessert die Wirksamkeit. Als Alternative zum Hilfsstoff Tween^{®}20 kann auch Polypropylenglykol als Hilfsstoff verwendet werden.

### Beispiel 38: λ-Carrageenan mit Blut

Probenmaterial: Rattenblut
Werkzeugbeschreibung (Art und Menge von Wirk- und Hilfsstoffen): Kunststoffzahnstocher beschichtet mit 1 % λ-Carrageenan (CAS 9064-57-7) in 10 %iger Tween^{®}20-Lösung
Primer: Primerpaar # 3
PCR-Programm: # 17
Das Ergebnis ist in Figur 38 gezeigt: M: Marker - DNA-Leiter; A: Zahnstocher beschichtet mit Heparin in 10 %iger Tween^{®}20-Lösung; B: Zahnstocher beschichtet mit einer Lösung aus 1 % λ-Carrageenan, 10 % Tween^{®}20; C: Zahnstocher unbeschichtet; D: PCR-Negativkontrolle (no template control)
Fazit: Als Alternative zu Heparin kann auch λ-Carrageenan als Wirkstoff zur Beschichtung von Probentransferwerkzeugen verwendet werden, so dass bei Transfer von Rattenblut in die PCR mittels Carrageenan-beschichtetem Werkzeug ein Amplifikat der Zielgröße (191 bp) entsteht.

### Beispiel 39: λ-Carrageenan mit Fleisch (Ziege)

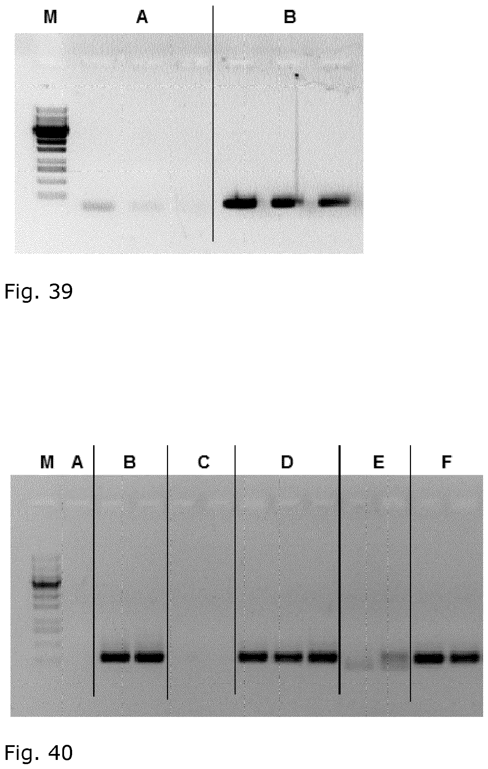

Probenmaterial: Ziegenfleisch (roh)
Werkzeugbeschreibung (Art und Menge von Wirk- und Hilfsstoffen): Kunststoffzahnstocher, der mit einer Lösung aus 0,5 % λ-Carrageenan, 10 % Tween^{®}20 beschichtet wurde.
Primer: Primerpaar # 16
PCR-Programm: # 18
Das Ergebnis ist in Figur 39 gezeigt: M: Marker, DNA-Leiter; A: Unbehandelter Zahnstocher zum Probentransfer; B: Zahnstocher wurde vor Probentransfer mit 0,5 % λ-Carrageenan, 10 % Tween^{®}20 beschichtet.
Fazit: Wird mittels eines mit λ-Carrageenan-Tween^{®}20 behandelten Zahnstochers Probenmaterial von Ziegenrohfleisch durch Anstechen aufgenommen und in die PCR transferiert, kann das Zielfragment von 142 bp amplifiziert werden, wohingegen bei Verwendung eines unbeschichteten Zahnstochers kein oder viel weniger Amplifikat gebildet wird.

### Beispielgruppe V: Unterschiedliche Materialien und Formen des Probentransferwerkzeugs

### Beispiel 40: Zahnstocher, Pipettenspitzen, Büroklammer

Probenmaterial: Baumwollblätter
Werkzeugbeschreibung (Art und Menge von Wirk- und Hilfsstoffen): Kunststoffzahnstocher sowie Pipettenspitze 0,5-10 µl, Büroklammer behandelt mit 38 IE Heparin/ml 10 %iger Tween^{®}20 Lösung
Primer: Primerpaar # 5
PCR-Programm: # 1
Das Ergebnis ist in Figur 40 gezeigt: M: Marker - DNA-Leiter; A: Kunststoffzahnstocher unbeschichtet; B: Kunststoffzahnstocher beschichtet mit Heparin/Tween; C: Pipettenspitze unbeschichtet; D: Pipettenspitzen beschichtet mit Heparin/Tween; E: Draht (Büroklammer, aufgebogen) unbeschichtet; F: Draht (Büroklammer, aufgebogen) beschichtet mit Heparin/Tween^{®}
Fazit: Nicht nur Kunststoffzahnstocher können durch Beschichtung mit einem Wirkstoff in Inhibitor inaktivierende Probentransferwerkzeuge überführt werden, sondern auch hohle, röhrenförmige Pipettenspitzen sowie Draht (aufgebogene Büroklammer).

### Beispiel 41: Combitips Innenteil, Büroklammer mit Baumwollblatt

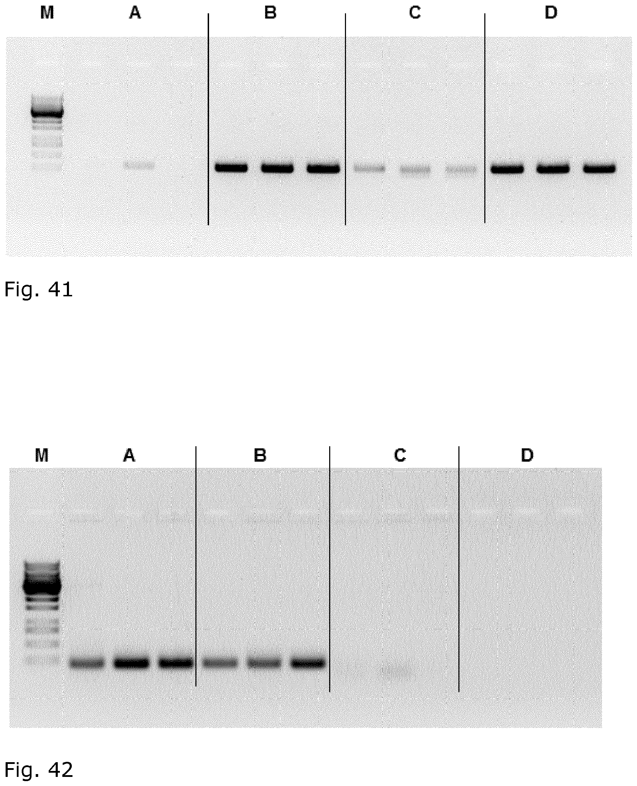

Probenmaterial: Baumwollblätter
Werkzeugbeschreibung (Art und Menge von Wirk- und Hilfsstoffen): Kunststoff-stäbchen (Innenteil einer Combitip 100 µl), Draht (Büroklammer) behandelt mit 38 IE Heparin/ml 10 %iger Tween^{®}20 Lösung
Primer: Primerpaar # 5
PCR-Programm: # 12
Das Ergebnis ist in Figur 41 gezeigt: M: Marker - DNA-Leiter; A: Innenteil einer Combitip unbeschichtet; B: Innenteil einer Combitip beschichtet; C: Draht (Büroklammer) unbeschichtet; D: Draht (Büroklammer) beschichtet
Fazit: Durch Beschichtung von Kunststoff-Stäbchen (Innenteil einer Combitip Pipettenspitze) die keine Zahnstocher sind, sowie einer aufgebogenen Büroklammer mit Heparin/Tween^{®}20 kann ein Inhibitor-inkativierendes Probentransferwerkzeug hergestellt werden.

### Beispiel 42: Polystyrolstäbchen aus Wägeschiffchen mit Blut

Probenmaterial: Rattenblut
Werkzeugbeschreibung (Art und Menge von Wirk- und Hilfsstoffen): Kunststoffzahnstocher und ein Abschnitt (spitz zugeschnitten) eines Polystyrol-Wägeschälchens behandelt mit 38 IE Heparin/ml 10 %iger Tween^{®}20 Lösung
Primer: Primerpaar # 3
PCR-Programm: # 17
Das Ergebnis ist in Figur 42 gezeigt: M: Marker - DNA-Leiter; A: Zahnstocher beschichtet mit Heparin/Tween^{®}20; B: Spitz zugeschnittener Abschnitt eines Polystyrol Wägeschälches beschichtet mit Heparin/Tween^{®}20; C: Zahnstocher (unbeschichtet); D: Spitz zugeschnittener Abschnitt eines Polystyrol Wägeschälches (unbeschichtet)
Fazit: Als Werkzeugmaterial kommt neben Kunststoffen, die typischerweise für Zahnstocher verwendet werden, auch spitz zulaufende Stäbchen aus Polystyrolmaterial zur Herstellung von Inhibitor inaktivierenden Probentransferwerkzeuge in Frage.

### Beispiel 43: Pipettenspitze mit Blut

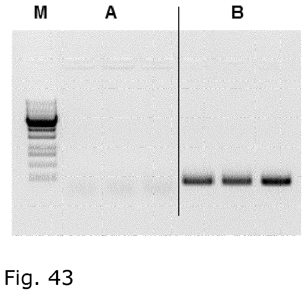

Probenmaterial: Rattenblut
Werkzeugbeschreibung (Art und Menge von Wirk- und Hilfsstoffen): Labortypische 10/20 µl Pipettenspitze (vor Firma Starlab) beschichtet mit 38 IE Heparin/ml 10 %iger Tween^{®}20 Lösung
Primer: Primerpaar # 3
PCR-Programm: # 17
Das Ergebnis ist in Figur 43 gezeigt: M: Marker, DNA-Leiter; A: Unbeschichtete Pipettenspitze; B: Beschichtete Pipettenspitze
Fazit: Auch labortypische Pipettenspitzen können durch Beschichtung mit Heparin/Tween^{®}20 in einen Inhibitor inaktivierendes Probentransferwerkzeug überführt werden.

### Beispiel 44: Ermittlung der Wirkstoffmengen am Transfer Tool.

A. Getestet und als wirksam festgestellte Wirkstoffkonzentrationen der Beschichtungslösung.

| Beispiel | Wirkstoffkonzentration in der Beschichtungslösung mit positiven Ergebnissen |
|---|---|
| 1 | 24 IE, 240 IE Heparin / ml |
| 2 | 240 I.E. Heparin / ml |
| 3 | 40 I.E. Heparin / ml |
| 4 | 40 I.E. Heparin / ml |
| 5 | 40 I.E. Heparin / ml |
| 6 | 38 I.E. Heparin / ml |
| 7 | 38 I.E. Heparin / ml |
| 8 | 38 I.E. Heaprin / ml |
| 9 | 38 IE Heparin / ml |
| 10 | 40 IE Heparin / ml |
| 11 | 38 IE Heparin / ml |
| 12 | 38 IE Heaprin / ml |
| 13 - 28 | 38 IE Heparin / ml |
| 29 | 1% und 5% Chondrotinsulfat-Natriumsalz |
| 30 | 5% Chondrotinsulfat-Natriumsalz, 1%, 0,5%, 0,1% Dextransulfat, |
| 31 | 6 M GITC, 90 mM EDTA, 1,5% Tewen^{®}, 50 mM Tris pH8 |
| 32 | 0,5%, 0,1%, 0,05% Carrageenan |
| 33 | 0,05%, 0,01% Dextransulfat |
| 34 | 0,01% Dextransulfat |
| 35 | 0,01%, 0,005% Dextransulfat |
| 36 | 4 M, 5 M GITC, |
| | 6,4 M, 8 M GuHCl |
| 37 | Heparin |
| 38 | 1% Carrageenan |
| 39 | 0,5% Carrageenan |
| 40 | 38 IE Heparin |
| 41 | 38 IE Heparin |
| 42 | 38 IE Heparin |
| 43 | 38 IE Heparin |

Wirksame Wirkstoffkonzentrationsbereiche der Beschichtungslösungen:
Heparin: 24 - 240 IE / ml
Chondrotinsulfat-Natriumsalz: 1% - 5%
Dextransulfat: 0,005 - 1%
Carrageenan: 0,05% - 1%
Chaotropsalz: 4 M - 8 M

B. Flüssigkeitsmenge, die von einem Zahnstocher durch Eintauchen von ca. 1 cm der Spitze aufgenommen wird.

Die auf der Zahnstocherspitze nach Eintauchen haftende Flüssigkeitsmenge wurde zunächst auf 5 nl geschätzt (aufgrund von Messungen mit Tinte), dies erschien aber recht wenig, daher erfolgte eine direkte Messung mit 200 Zahnstochern.

Gemessene Menge an Beschichtungslösung, die bei 10 mm Eintauchtiefe am Zahnstocher haften bleibt: ca. 0,132 µl pro Zahnstocher (Mittel von 200 Zahnstochern).

C. Ermittlung der Oberfläche der Zahnstocherspitze für Eintauchtiefe von 10 mm.
Druchmesser auf 10 mm Höhe: 1,0 - 1,4. Mittel: 1,2 mm
Durchmesser auf 1 mm Höhe: 0,54 - 0,60 mm. Mittel: 0,57 mm.
Mittlerer Durchmesser: 0,885 mm
Mittlerer Umfang: 2Pi*r=0,885*3,14=2,78 mm
Zylinderfläche: U*h = 2,78 * 10 = 27,8 mm².
Spitzenfläche: F=Pir² = (0,57/2)² * 3,14 = 0,255 mm²
Oberfläche Zahnstocher bei Eintauchtiefe von 10 mm: 27,8 + 0,895 = 28,1 mm² Gemessene Menge an Beschichtungslösung, die bei 10 mm Eintauchtiefe am Zahnstocher haften bleibt: ca. 0,132 µl pro Zahnstocher (Mittel von 200 Zahnstochern).

D. Berechnung der Wirkstoffmenge je mm² beschichteter Zahnstocheroberfläche. Heparin:
24 - 240 IE / ml --> Je 0,132 µl anhaftender Beschichtungslösung sind das 0,003 - 0,03 IE Heparin.
Verteilt auf 28,1 mm², ergibt sich: 0,0001 - 0,001 IE Heparin/mm² bzw. 0,01 - 0,1 IE Heparin/cm².
Fazit: Die beschichtete Oberfläche des Zahnstochers mit Heparin als Wirkstoff weist eine Heparinmenge von ca. 0,01 - 0,1 IE Heparin/cm² auf.

Chondrotinsulfat-Natriumsalz:
1% - 5% --> 1 - 5 g/100 ml --> 0,01 - 0,05 g/ml --> 10 - 50 mg/ml --> 0,01 - 0,05 mg/µl.
Je 0,132 µl Anhaftungslösung sind das 0,0013 - 0,0066 mg Wirkstoff am Zahnstocher.
Verteilt sich diese Menge auf 28,1 mm², ergibt sich: 0,000046 - 0,00023 mg/mm², oder 0,0046 - 0,023 mg/cm² , oder 4,6 - 23 µg/cm².

Dextransulfat:
0,005% - 1% --> 0,005 - 1 g / 100 ml --> 0,00005 - 0,01 g/ml oder mg/pL --> 0,05 - 10 µg/µl
Je 0,132 µl Anhaftungslösung sind das 0,0066 - 1,3 µg Wirkstoff am Zahnstocher. Verteilt sich diese Menge auf 28,1 mm², ergibt sich: 0,00023 - 0,046 µg/mm², oder 0,023 - 4,6 µg/cm².

Carrageenan:
0,05% - 1% in der Wirkstofflösung, oder 0,23 - 4,6 µg/cm².

Chaotropsalz:
4 M - 8 M --> 4 - 8 mol/l oder µmol/µl
Je 0,132 µl Anhaftungslösung sind das 0,53 - 1,1 µmol Wirkstoff am Zahnstocher. Verteilt sich diese Menge auf 28,7 mm², ergibt sich: 0,018 - 0,037 µmol/mm², oder 1,8 - 3,7 µmol/cm².
E. Fazit.
Heparin: 0,01 - 0,1 IE je cm² Stäbchenoberfläche
Andere Polysaccaride: 0,023 - 23 µg/cm². Gerundet 0,02 - 25 µg/cm²
Chaotropsalz: 1,8 - 3,7 µmol/cm². Gerundet 1,5 - 4 µmol/cm².

## Patentansprüche

1. Verwendung eines stabförmigen Probentransferwerkzeugs zum direkten Transfer von kleinen Nukleinsäure-haltigen Proben in eine enzymatische Reaktion, wobei das Probentransferwerkzeug so dimensioniert ist, dass es zum direkten Transfer von Probenvolumen kleiner als 1 µl oder Probenmengen von 10 bis 500 µg geeignet ist, und das einen Bereich aufweist, der zum Kontakt mit der Nukleinsäure-haltigen Probe bestimmt ist und der mit einem oder mehreren Wirkstoffen beschichtet ist, der/die in einer nach einem Transfer erfolgenden enzymatische Reaktion diejenigen in der Nukleinsäure-haltigen Probe enthaltenen Stoffe, inhibiert(en), inaktiviert(en) oder deren inhibitorische Wirkung reduziert(en), die die nach dem Transfer erfolgende enzymatische Reaktion hemmen, in dem diese in der Probe enthaltenen Stoffe zumindest teilweise gebunden oder inaktiviert werden.

2. Verwendung nach Anspruch 1, wobei
(i) das Probentransferwerkzeug geeignet ist, entweder durch den/die Wirkstoff(e) diese Verbindungen/Inhibitoren zu binden oder den/die Wirkstoff(e) abzugeben, um dadurch die Inhibitoren zu inaktivieren; und/oder
(ii) der/die Wirkstoffe ausgewählt sind aus Antikoagulantien und Polysacchariden wie Heparin, Chondrotinsulfat, Heparansulfat, Keratansulfat, Amidopektinen, Hyaluronsäure, Pektinen, Xanthan, Chitosan, Oligo-Glucosamin, Dextransulfat, Carrageen, Methylcellulose, Guaran und anderen Stoffen wie Chaotropsalze, für die bekannt ist, dass sie enzymatische Reaktionen hemmende/inhibitorische Stoffe inaktivieren; und/oder
(iii) der/die Wirkstoffe bei Kontakt mit der Probe in die Probe abgegeben oder in der Probe gelöst werden.

3. Verwendung nach Anspruch 1 oder 2, wobei der/die Wirkstoffe in einer ausreichenden Inhibitor-hemmenden Menge auf dem Probentransferwerkzeug vorhanden ist/sind, wobei insbesondere für Heparin eine Beschichtung von 0,001 bis 0,5, bevorzugt 0,01 bis 0,1 IE/cm² Probentransferwerkzeugoberfläche, für andere Polysaccharide eine Beschichtung von 0,005 bis 500, bevorzugt 0,02 bis 25 µg/cm² Probentransferoberfläche und für Chaotropsalze eine Beschichtung von 0,1 bis 50, bevorzugt 1,5 bis 4 µmol/cm² Probentransferwerkzeugoberfläche auf dem Probentransferwerkzeug vorhanden ist/sind.

4. Verwendung nach einem oder mehreren der Ansprüche 1 bis 3, wobei das Probenwerkzeug
(i) die Form eines Stäbchens, eines Stiftes oder einer Nadel aufweist; und/oder
(ii) aus einem festen Material ausgewählt aus Kunststoff, Holz, Pappe, Papier, Keramik, Glas, Keramik, Ton, Magnesia, Metall und Kombinationen daraus besteht; und/oder
(iii) weiterhin einen Bereich mit dem das Probentransferwerkzeug durch den Operator, einschließlich einem Menschen oder einer Maschine, gehalten wird, umfasst; und/oder
(iv) so dimensioniert ist, dass es zum Transferieren von Probenvolumen 10 bis 500 nl, bzw. von Probenmengen von 10 bis 500 µg, vorzugsweise von Probenvolumen von 50 bis 200 nl, bzw. von Probenmenge von 50 bis 200 µg, geeignet ist; und/oder
(v) so mit dem/den Wirkstoff(en) beschichtet ist, dass eine ausreichende Inhibitor-hemmenden Menge des/der Wirkstoffe auf dem Probentransferwerkzeug vorhanden ist/sind; und/oder
(vi) neben dem/den Wirkstoffen noch mit weitere neutralen Detergenzien und Salzen beschichtet ist.

5. Verwendung nach einem oder mehreren der Ansprüche 1 bis 4, wobei das Probentransferwerkzeug aus einem Plastikstäbchen, Zahnstocher oder Kunststoffzahnstocher besteht, das/der mit Heparin beschichtet ist.

6. Verwendung nach einem oder mehreren der Ansprüche 1 bis 5, wobei der Transfer der Nukleinsäure-haltigen Probe durch In-Kontakt-bringen des Probentransferwerkzeugs mit einer festen oder flüssigen Phase der Nukleinsäure-haltigen Probe, so dass eine ausreichende Menge der Nukleinsäure-haltigen Probe an dem Probentransferwerkzeug haften bleibt, und nachfolgendes Eintauchen des Probentransferwerkzeugs in das Reaktionsgemisch erfolgt.

7. Verwendung nach einem oder mehreren der Ansprüche 1 bis 6, wobei die enzymatische Reaktion eine enzymatische Nachweisreaktion ist, die insbesondere ausgewählt ist aus PCR, Reverser Transkription, isothermaler Amplifikation, Restriktion, Sequenzierung und Nukleinsäureamplifikation.

8. Verwendung nach einem oder mehreren der Ansprüche 1 bis 7, wobei die Proben ausgewählt sind aus Körperbestandteilen und Körperflüssigkeiten, einschließlich Blut, Urin, Speichel, Zellen, Gewebe und Stuhl, Pflanzen und Pflanzenbestandteilen, einschließlich jeglicher Art von Pflanzenmaterial, wobei Körperflüssigkeiten, Pflanzenmaterial und Stuhlproben besonders bevorzugt sind.

9. Nachweisverfahren umfassend:
(a) in-Kontakt-bringen von einem stabförmigen Probentransferwerkzeug, das so dimensioniert ist, dass es zum direkten Transfer von Probenvolumen kleiner als 1 µl oder Probenmengen von 10 bis 500 µg geeignet ist, und das einen Bereich aufweist, der zum Kontakt mit einer Nukleinsäure-haltigen Probe bestimmt ist und der mit einem oder mehreren Wirkstoffen beschichtet ist, der/die in einer nach einem Transfer erfolgenden enzymatische Reaktion diejenigen in der Nukleinsäure-haltigen Probe enthaltenen Stoffe, inhibiert(en), inaktiviert(en) oder deren inhibitorische Wirkung reduziert(en), die die nach dem Transfer erfolgende enzymatische Reaktion hemmen, in dem diese in der Probe enthaltenen Stoffe zumindest teilweise gebunden oder inaktiviert werden, mit einer festen oder flüssigen Phase der Nukleinsäure-haltigen Probe, so dass eine ausreichende Menge der Nukleinsäure-haltigen Probe an dem Probentransferwerkzeug haften bleibt,
(b) direkten Transfer des Probentransferwerkzeugs zu einem Reaktionsgemisch für eine enzymatische Nachweisreaktion und Eintauchen des Probentransferwerkzeugs in das Reaktionsgemisch, so dass eine für die Nachweisreaktion hinreichende Menge der Nukleinsäure-haltigen Probe in die Enzymreaktion eingetragen wird,
(c) Entfernen des Probentransferwerkzeug aus dem Reaktionsgemisch für die enzymatische Nachweisreaktion; und
(d) Durchführung der enzymatischen Nachweisreaktion.

10. Nachweisverfahren nach Anspruch 9, wobei das Probentransferwerkzeug ein wie in Ansprüchen 2 bis 5 definiertes Probenwerkzeug ist.

11. Nachweisverfahren nach Anspruch 9 oder 10, wobei
(i) die enzymatische Nachweisreaktion ausgewählt ist aus PCR, Reverser Transkription, isothermaler Amplifikation, Restriktion, Sequenzierung und Nukleinsäureamplifikation; und/oder
(ii) die Proben ausgewählt sind aus Körperbestandteilen und Körperflüssigkeiten, einschließlich Blut, Urin, Speichel, Zellen, Gewebe und Stuhl, Pflanzen und Pflanzenbestandteilen, einschließlich jeglicher Art von Pflanzenmaterial, wobei Körperflüssigkeiten, Pflanzenmaterial und Stuhlproben besonders bevorzugt sind; und/oder
(iii) das Reaktionsgemisch sich in einem Reaktionsgefäß, in einem Napf einer Titerplatte oder auf einer ebenen Platte befindet.

12. Nachweisverfahren nach einem oder mehreren der Ansprüche 9 bis 11, wobei
(i) Schritte (a) und (b), unabhängig voneinander, manuell durch einen Operator, oder maschinell durchgeführt werden; und/oder
(ii) in Schritten (a) und (b) eine kleines Probenvolumen von weniger als 1 µl oder ein Probenvolumen von 10 bis 500 nl, vorzugsweise von 50 bis 200 nl, bzw. eine Probenmenge von 10 -500 µg, vorzugsweise von 50 - 200 µg transferiert wird; und/oder
(iii) das Reaktionsvolumen der Nachweisreaktion 1 bis 100 µl, vorzugsweise etwa 10 µl beträgt; und/oder
(iv) vor dem in-Kontakt-bringen von Schritt (a) eine Lyse des Probenmaterials erfolgt.

13. Verwendung eines Kits zur Durchführung des Nachweisverfahrens nach einem oder mehreren der Ansprüche 9 bis 12, wobei der Kit ein oder mehrere stabförmige Probentransferwerkzeuge umfasst, die so dimensioniert sind, dass sie zum direkten Transfer von Probenvolumen kleiner als 1 µl oder Probenmengen von 10 bis 500 µg geeignet sind, und die einen Bereich aufweisen, der zum Kontakt mit einer Nukleinsäure-haltigen Probe bestimmt ist und der mit einem oder mehreren Wirkstoffen beschichtet ist, der/die in einer nach einem Transfer erfolgenden enzymatische Reaktion diejenigen in der Nukleinsäure-haltigen Probe enthaltenen Stoffe, inhibiert(en), inaktiviert(en) oder deren inhibitorische Wirkung reduziert(en), die die nach dem Transfer erfolgende enzymatische Reaktion hemmen, in dem diese in der Probe enthaltenen Stoffe zumindest teilweise gebunden oder inaktiviert werden.

14. Verwendung nach Anspruch 13, wobei der Kit weiterhin Reagenzien, Reaktionsgemische und/oder Lösungsmittel für die Nachweisreaktion, und/oder Reagenzien für die Lyse des Probenmaterials umfasst.

## Claims

1. Use of a rod-shaped sample transfer tool for the direct transfer of small samples containing nucleic acids into an enzymatic reaction, wherein said sample transfer tool is sized to be suitable for the direct transfer sample volumes of smaller than 1 µg, or sample amounts of from 10 to 500 µg, and includes a region designed for contact with the sample containing the nucleic acid which is coated with one or more active substances that, in an enzymatic reaction taking place after a transfer, inhibit, inactivate, or reduce the inhibitors effect of, those substances contained in said sample containing nucleic acids that inhibit the enzymatic reaction taking place after the transfer, by at least partially binding or inactivating such substances contained in the sample.

2. The use according to claim 1, wherein
(i) said sample transfer tool is suitable for either binding said compounds/inhibitors by the active substance or substances, or releasing the active substance or substances to thereby inactivate the inhibitors; and/or
(ii) said active substance or substances are selected from anticoagulants and polysaccharides, such as heparin, chondroitin sulfate, heparan sulfate, keratan sulfate, amidopectins, hyaluronic acid, pectins, xanthan, chitosan, oligo-glucosamine, dextran sulfate, carrageenan, methylcellulose, guaran and other materials, such as chaotropic salts which are known to inactivate materials inhibiting enzymatic reactions; and/or
(iii) said active substance or substances are released into or dissolved in the sample upon contact with the sample.

3. The use according to claim 1 or 2, wherein said active substance or substances are present on said sample transfer tool in a sufficient inhibitor-inhibiting amount, in which a coating of 0.001 bis 0.5, preferably 0.01 to 0.1, IU/cm² of sample transfer tool surface especially for heparin, a coating of 0.005 to 500, preferably from 0.02 to 25 µg/cm² of sample transfer surface, for other polysaccharides, and a coating of 0.1 to 50, preferably 1.5 to 4 µmol/cm² of sample transfer tool surface for chaotropic salts is present on said sample transfer tool.

4. The use according to one or more of claims 1 to 3, in which said sample tool
(i) has the shape of a rod, a pin, a needle, a tube or a pipette tip; and/or
(ii) is made of a solid material selected from plastic, wood, cardboard, paper, ceramics, glass, ceramics, clay, magnesia, metal, and combinations thereof; and/or
(iii) further includes a region at which the sample transfer tool is held by the operator, including a human or a machine; and/or
(iv) is sized to be able to transfer sample volumes of from 10 to 500 nl, or sample quantities of from 10 to 500 µg, preferably sample volumes of from 50 to 200 µl, or sample quantities of from 50 to 200 µg; and/or
(v) is coated with said active substance(s) in such a way that a sufficient inhibitor-inhibiting amount of said active substance(s) is present on the sample transfer tool; and/or
(vi) is coated with other neutral surfactants and salts in addition to said active substance or substances.

5. The use according to one or more of claims 1 to 4, wherein said sample transfer tool consists of a plastic rod, toothpick or plastic toothpick coated with heparin.

6. The use according to one or more of claims 1 to 5, wherein said transfer of the sample containing nucleic acids is effected by contacting the sample transfer tool with a solid or liquid phase of the sample containing nucleic acids, so that a sufficient amount of said sample containing nucleic acids becomes adhered to said sample transfer tool, followed by immersing the sample transfer tool into the reaction mixture.

7. The use according to one or more of claims 1 to 6, wherein said enzymatic reaction is an enzymatic detection reaction, in particular, one selected from PCR, reverse transcription, isothermal amplification, restriction digestion, sequencing and nucleic acid amplification.

8. The use according to one or more of claims 1 to 7, wherein the samples are selected from body components and body fluids including blood, urine, saliva, cells, tissues and stool, plants and plant components including any kind of plant material, in which body fluids, plant material and stool specimens are particularly preferred.

9. A detection method, comprising:
(a) contacting a rod-shaped sample transfer tool sized to be suitable for the direct transfer sample volumes of smaller than 1 µl, or sample amounts of from 10 to 500 µg, and includes a region designed for contact with the sample containing the nucleic acid which is coated with one or more active substances that, in an enzymatic reaction taking place after a transfer, inhibit, inactivate, or reduce the inhibitors effect of, those substances contained in said sample containing nucleic acids that inhibit the enzymatic reaction taking place after the transfer, by at least partially binding or inactivating such substances contained in the sample with a solid or liquid phase of a sample containing nucleic acids, so that a sufficient amount of said sample containing nucleic acids becomes adhered to said sample transfer tool;
(b) direct transfer of the sample transfer tool to a reaction mixture for an enzymatic detection reaction, and immersing the sample transfer tool into the reaction mixture, so that an amount of said sample containing nucleic acids sufficient for the detection reaction is carried into the enzyme reaction;
(c) removing the sample transfer tool from the reaction mixture for said enzymatic detection reaction; and
(d) performing said enzymatic detection reaction.

10. The detection method according to claim 9, wherein said sample transfer tool is a sample transfer tool as defined in claims 2 to 5.

11. The detection method according to claim 9 or 10, wherein
(i) the enzymatic detection reaction is selected from PCR, reverse transcription, isothermal amplification, restriction digestion, sequencing and nucleic acid amplification; and/or
(ii) the samples are selected from body components and body fluids including blood, urine, saliva, cells, tissues and stool, plants and plant components including any kind of plant material, in which body fluids, plant material and stool specimens are particularly preferred; and/or
(iii) said reaction mixture is present in a reaction vessel, in a well of a titration plate, or on a planar plate.

12. The detection method according to one or more of claims 9 to 11, wherein
(i) steps (a) and (b) are performed independently of one another, manually by an operator, or by a machine; and/or
(ii) a small sample volume of less than 1 µl, or a sample volume of from 10 to 500 nl, preferably from 50 to 200 nl, or a sample quantity of 10 to 500 µg, preferably 50 to 200 µg, is transferred in steps (a) and (b); and/or
(iii) the reaction volume of the detection reaction is from 1 to 100 µl, preferably about 10 µl; and/or
(iv) lysis of the sample material is performed before said contacting of step (a).

13. Use of a kit for performing the detection process according to claims 9 to 12, wherein said kit includes one or more rod-shaped sample transfer tools sized to be suitable for the direct transfer sample volumes of smaller than 1 µg, or sample amounts of from 10 to 500 µg, and includes a region designed for contact with the sample containing the nucleic acid which is coated with one or more active substances that, in an enzymatic reaction taking place after a transfer, inhibit, inactivate, or reduce the inhibitors effect of, those substances contained in said sample containing nucleic acids that inhibit the enzymatic reaction taking place after the transfer, by at least partially binding or inactivating such substances contained in the sample.

14. The use according to claim 13, wherein said kit further comprises reagents, reaction mixtures and/or solvents for the detection reaction, and/or reagents for the lysis of the sample material.

## Revendications

1. Utilisation d'un outil de transfert d'échantillon en forme de bâtonnet pour un transfert direct de petits échantillons contenant des acides nucléiques dans une réaction enzymatique, l'outil de transfert d'échantillons étant dimensionné de sorte qu'il soit adapté pour un transfert direct de volumes d'échantillon inférieurs à 1 µl ou de quantités d'échantillon de 10 à 500 µg, et qui comprend une zone destinée à venir en contact avec l'échantillon contenant des acides nucléiques et qui est revêtue d'un ou de plusieurs principes actifs qui, dans une réaction enzymatique postérieure à un transfert, inhibe(nt) ou rend(ent) inactive(s) celles des substances contenues dans l'échantillon contenant des acides nucléiques, ou en rédui(sen)t leur action inhibitrice, qui empêchent la réaction enzymatique postérieure au transfert, en liant au moins partiellement ces substances contenues dans l'échantillon ou en les rendant inactives.

2. Utilisation selon la revendication 1,
(i) l'outil de transfert d'échantillon étant adapté pour lier ces composés/inhibiteurs par le/les principe(s) actif(s) ou de libérer le/les principe(s) actif(s) afin d'inactiver les inhibiteurs ; et/ou
(ii) le(s) principe(s) actif(s) étant choisi(s) parmi des anticoagulants et des polysaccharides tels que l'héparine, le sulfate de chondroïtine, le sulfate d'héparane, le sulfate de kératane, les amidopectines, l'acide hyaluronique, les pectines, la gomme xanthane, le chitosane, l'oligo-glucosamine, le dextransulfate, la carraghénine, la méthylcellulose, le guarana et d'autres substances telles que les sels chaotropiques, qui sont connues pour inactiver les substances inhibitrices/empêchant des réactions enzymatiques ; et/ou
(iii) le/les principe(s) actif(s) étant libéré(s) dans l'échantillon ou dissouts dans l'échantillon au contact de l'échantillon.

3. Utilisation selon la revendication 1 ou 2, le(s) principe(s) actif(s) étant présent(s) sur l'outil de transfert d'échantillon en une quantité suffisante pour empêcher l'inhibiteur, en particulier un revêtement de 0,001 à 0,5, de préférence de 0,01 à 0,1 IE/cm² de surface d'outil de transfert d'échantillon pour l'héparine, un revêtement de 0,005 à 500, de préférence de 0,02 à 25 µg/cm² de surface de transfert d'échantillon pour d'autres polysaccharides, et un revêtement de 0,1 à 50, de préférence de 1,5 à 4 µmol/cm² de surface d'outil de transfert d'échantillon pour les sels chaotropiques étant présent(s) sur l'outil de transfert d'échantillon.

4. Utilisation selon l'une quelconque ou plusieurs des revendications 1 à 3, l'outil d'échantillon
(i) présentant la forme d'un bâtonnet, d'une broche ou d'une aiguille ; et/ou
(ii) étant constitué d'un matériau solide choisi parmi le plastique, le bois, le carton, le papier, la céramique, le verre, la céramique, l'argile, la magnésie, le métal et des combinaisons de ceux-ci ; et/ou
(iii) comprenant en outre une zone par laquelle l'outil de transfert d'échantillon est maintenu par l'opérateur, notamment un humain ou une machine ; et/ou
(iv) étant dimensionné de manière à pouvoir transférer un volume d'échantillon de 10 à 500 nl, ou un volume d'échantillon de 10 à 500 µg, de préférence un volume d'échantillon de 50 à 200 nl, ou un volume d'échantillon de 50 à 200 µg ; et/ou
(v) étant revêtu du/des principe(s) actif(s) de manière à ce qu'une quantité suffisante du/des principe(s) actif(s) pour empêcher l'inhibiteur soit/soient présente(s) sur l'outil de transfert d'échantillon ; et/ou
(vi) en plus du/des principe(s) actif(s), étant revêtu d'autres détergents et sels neutres.

5. Utilisation selon une ou plusieurs des revendications 1 à 4, l'outil de transfert d'échantillon étant constitué d'un bâtonnet en plastique, d'un cure-dent ou d'un cure-dent en plastique revêtu d'héparine.

6. Utilisation selon une ou plusieurs des revendications 1 à 5, le transfert de l'échantillon contenant des acides nucléiques étant effectué par mise en contact de l'outil de transfert d'échantillon avec une phase solide ou liquide de l'échantillon contenant des acides nucléiques de sorte qu'une quantité suffisante de l'échantillon contenant des acides nucléiques adhère à l'outil de transfert d'échantillon, et puis une immersion de l'outil de transfert d'échantillon dans le mélange réactionnel.

7. Utilisation selon une ou plusieurs des revendications 1 à 6, la réaction enzymatique étant une réaction de détection enzymatique qui est choisie en particulier parmi la PCR, la transcription inverse, l'amplification isotherme, la restriction, le séquençage et l'amplification d'acide nucléique.

8. Utilisation selon une ou plusieurs des revendications 1 à 7, les échantillons étant choisis parmi des composants corporels et des fluides corporels, notamment le sang, l'urine, la salive, les cellules, les tissus et les selles, des plantes et des composants végétaux, y compris tout type de matière végétale, les fluides corporels, la matière végétale et les échantillons de selles étant particulièrement préférés.

9. Procédé de détection, comprenant les étapes consistant à :
(a) mettre en contact un outil de transfert d'échantillon en forme de bâtonnet, qui est dimensionné pour être adapté pour transférer directement des volumes d'échantillon inférieurs à 1 µl ou des quantités d'échantillon de 10 à 500 µg et qui comprend une zone destinée à être en contact avec un échantillon contenant un acide nucléique et qui est revêtu d'un ou de plusieurs principes actifs qui, dans une réaction enzymatique postérieure au transfert, inhibe(nt) ou rend(ent) inactive(s) celles des substances contenues dans l'échantillon contenant des acides nucléiques, ou en rédui(sen)t leur action inhibitrice, qui empêchent la réaction enzymatique postérieure au transfert, en liant au moins partiellement ces substances contenues dans l'échantillon ou en les rendant inactives, avec une phase solide ou liquide de l'échantillon contenant un acide nucléique, de sorte qu'une quantité suffisante de l'échantillon contenant un acide nucléique adhère à l'outil de transfert d'échantillon ;
(b) transférer directement l'outil de transfert d'échantillon vers un mélange réactionnel pour une réaction de détection enzymatique et immerger l'outil de transfert d'échantillon dans le mélange réactionnel de sorte qu'une quantité suffisante de l'échantillon contenant des acides nucléiques pour la réaction de détection soit introduite dans la réaction enzymatique ;
(c) retirer l'outil de transfert d'échantillon du mélange réactionnel pour la réaction de détection enzymatique ; et
(d) effectuer la réaction de détection enzymatique.

10. Procédé de détection selon la revendication 9, l'outil de transfert d'échantillon étant un outil d'échantillon tel que défini dans les revendications 2 à 5.

11. Procédé de détection selon la revendication 9 ou 10,
(i) la réaction de détection enzymatique étant choisie parmi la PCR, la transcription inverse, l'amplification isotherme, la restriction, le séquençage et l'amplification d'acide nucléique ; et/ou
(ii) les échantillons étant choisis parmi des composants corporels et des fluides corporels, notamment le sang, l'urine, la salive, les cellules, les tissus et les selles, des plantes et des composants végétaux, y compris tout type de matière végétale, les fluides corporels, la matière végétale et les échantillons de selles étant particulièrement préférés ; et/ou
(iii) le mélange réactionnel se trouve dans un récipient de réaction (3), dans une coupelle d'une plaque de titre ou sur une plaque plane.

12. Procédé de réaction selon l'une quelconque ou plusieurs des revendications 9 à 11,
(i) les étapes (a) et (b) étant effectuées indépendamment l'une de l'autre, manuellement par un opérateur ou par une machine ; et/ou
(ii) dans les étapes (a) et (b), un petit volume d'échantillon inférieur à 1 µl ou un volume d'échantillon de 10 à 500 nl, de préférence de 50 à 200 nl, ou une quantité d'échantillon de 10 à 500 µg, de préférence de 50 à 200 µg, étant transféré(e) ; et/ou
(iii) le volume de réaction de la réaction de détection étant de 1 à 100 µl, de préférence d'environ 10 µl ; et/ou
(iv) une lyse du matériau d'échantillon étant effectuée avant la mise en contact de l'étape (a).

13. Utilisation d'un kit pour la mise en oeuvre du procédé de détection selon une ou plusieurs des revendications 9 à 12, le kit comprenant un ou plusieurs outils de transfert d'échantillon en forme de bâtonnet, qui sont dimensionnés de manière à être adaptés pour un transfert direct de volumes d'échantillon inférieurs à 1 µl ou de quantités d'échantillon de 10 à 500 µg, et qui comprennent une zone qui est destinée à entrer en contact avec un échantillon contenant un acide nucléique et qui est revêtu d'un ou de plusieurs principes actifs qui, dans une réaction enzymatique se produisant après le transfert, inhibe(nt) ou rend(ent) inactive(s) celles des substances contenues dans l'échantillon contenant des acides nucléiques, ou en rédui(sen)t leur action inhibitrice, qui empêchent la réaction enzymatique postérieure au transfert, en liant au moins partiellement ces substances contenues dans l'échantillon ou en les rendant inactives.

14. Utilisation selon la revendication 13, le kit comprenant en outre des réactifs, des mélanges réactionnels et/ou des solvants pour la réaction de détection, et/ou des réactifs pour la lyse du matériau d'échantillon.
